(19) Europäisches Patentamt
European Patent Office
Office européen des brevets

(11) EP 4 752 145 A1

(12) EUROPEAN PATENT APPLICATION
published in accordance with Art. 153(4) EPC

(43) Date of publication:
**03.06.2026 Bulletin 2026/23**

(21) Application number: **24844874.8**

(22) Date of filing: **26.07.2024**

(51) International Patent Classification (IPC):
***C07D 491/056*** (2006.01) ***C07D 471/04*** (2006.01)
***C07D 498/04*** (2006.01) ***C07D 401/02*** (2006.01)
***C07D 417/12*** (2006.01) ***C07D 487/04*** (2006.01)
***C07D 401/12*** (2006.01) ***C07D 403/12*** (2006.01)
***A61K 31/519*** (2006.01) ***A61K 31/444*** (2006.01)
***A61K 31/501*** (2006.01) ***A61K 31/505*** (2006.01)
***A61K 31/4745*** (2006.01) ***A61P 9/00*** (2006.01)
***A61P 3/06*** (2006.01) ***A61P 1/16*** (2006.01)
***A61P 9/10*** (2006.01)

(52) Cooperative Patent Classification (CPC):
**A61K 31/444; A61K 31/4745; A61K 31/501; A61K 31/505; A61K 31/519; A61P 1/16; A61P 3/06; A61P 9/00; A61P 9/10; C07D 401/02; C07D 401/12; C07D 403/12; C07D 417/12; C07D 471/04; C07D 487/04;** (Cont.)

(86) International application number:
**PCT/CN2024/107781**

(87) International publication number:
**WO 2025/021188 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.07.2023 CN 202310930420**
**25.12.2023 CN 202311791758**
**03.06.2024 CN 202410707101**
**14.06.2024 CN 202410766231**

(71) Applicant: **Tuojie Biotech (Shanghai) Co., Ltd.**
**Shanghai 201203 (CN)**

(72) Inventors:
- **HU, Tao**
  **Shanghai 201203 (CN)**
- **TAN, Liang**
  **Shanghai 201203 (CN)**
- **LIU, Haomiao**
  **Shanghai 201203 (CN)**
- **LI, Yunfei**
  **Shanghai 201203 (CN)**

(74) Representative: **Dragotti & Associati S.P.A.**
**Via Nino Bixio, 7**
**20129 Milano (MI) (IT)**

# (54) DIAMINOCYCLOPENTYL SUBSTITUTED HETEROARYL DERIVATIVES, USE THEREOF AND PREPARATION METHOD THEREFOR

(57) Provided are diaminocyclopentyl substituted heteroaryl derivatives, the use thereof and a preparation method therefor. In particular, the present disclosure relates to compounds represented by formula (I) each substituent of which is defined in the description, and a preparation method therefor. The compounds can be used as PCSK9 inhibitors to prevent and/or treat PCSK9-mediated diseases and disorders.

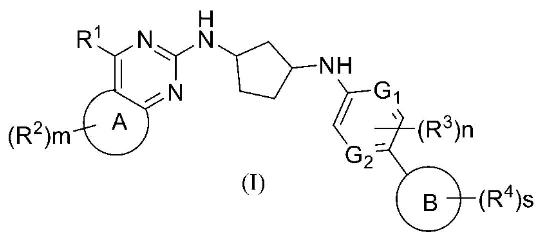

(I)

EP 4 752 145 A1

(52) Cooperative Patent Classification (CPC): (Cont.)
**C07D 491/056; C07D 498/04**

**Description**

**TECHNICAL FIELD**

The present disclosure relates to a diaminocyclopentyl-substituted heteroaryl derivative, use thereof, and a preparation method therefor, and pertains to the field of pharmaceuticals.

**BACKGROUND**

PCSK9, also known as "proprotein convertase subtilisin/kexin 9", is a member of the secretory proprotein convertase family and plays an important role in cholesterol metabolism. PCSK9 increases the level of circulating LDL cholesterol through the enhanced degradation of the LDL receptor independently of its catalytic activity. Secreted PCSK9 binds to the epidermal growth factor domain A (EGFA) of the LDL receptor (LDLR) on the cell surface, and the PCSK9/LDL receptor complex is internalized into endosomal/lysosomal compartments. The enhanced binding affinity of PCSK9 for the LDL receptor at the acidic pH of late endosomes/lysosomes reduces LDL receptor recycling and targets the LDL receptor for lysosomal degradation instead. Genetic association studies have demonstrated that loss-of-function mutations in PCSK9 are associated with low plasma LDL-C levels and a reduction in the incidence of adverse cardiovascular events.

Another biological pathway involving the action of PCSK9 on the LDL receptor is the onset of septic shock. Septic shock is an often fatal complication of a severe microbial infection (sepsis) that triggers an uncontrolled systemic inflammatory response and subsequent organ failure. Sepsis originates from microbial cell walls containing pathogenic lipid moieties, such as lipopolysaccharide (LPS; Gram-negative bacteria). LPS is an effective ligand for mammalian innate immune receptors [Toll-like receptors (TLRs)] and thus plays a prominent role in the septic inflammatory response (septic shock or sepsis). PCSK9 reduces LPS uptake through the LDL receptor in the liver, such that free LPS overstimulates the immune response of the body to pathogens that lead to sepsis. Thus, inhibiting PCSK9 is beneficial in retaining the liver LDL receptor to enable systemic pathogen clearance and detoxification in response to sepsis. However, apart from antibiotic therapy, there are currently no effective treatments for sepsis or septic shock.

Two monoclonal antibodies (mAbs) that selectively bind to extracellular PCSK9 and prevent its interaction with the LDL receptor, alirocumab and evolocumab, have recently been approved by the FDA. They can significantly lower LDL levels. However, mAb drugs require intravenous injection, resulting in poor patient compliance. Thus, ease of dosing and administration becomes a key factor to patient compliance with maintenance drug treatments. Published patent applications related to PCSK9 inhibitors include WO2023084449A, WO2020150473A, WO2020150474A, etc. There is a need for more PCSK9 inhibitors with increased efficacy and more ease of administration, which can be achieved through small-molecule PCSK9 inhibitors.

SUMMARY

The present disclosure provides a compound represented by formula (I) or a pharmaceutically acceptable salt thereof,

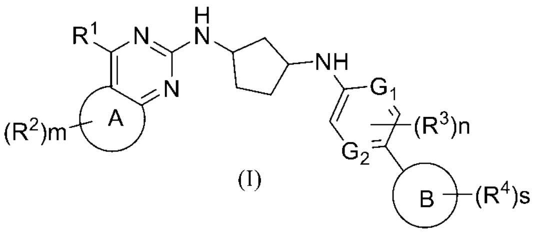

(I)

wherein:

$R^1$ is selected from the group consisting of hydrogen and halogen;
ring A is selected from the group consisting of 3- to 7-membered cycloalkyl and 3- to 7-membered heterocycloalkyl, and the heterocycloalkyl comprises at least one heteroatom selected from the group consisting of O, N, and S;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, and the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$;
or two $R^2$ attached to the same atom, together with the atom to which they are attached, form 3- to 6-membered cycloalkyl or 3- to 6-membered heterocycloalkyl, and the 3- to 6-membered cycloalkyl or 3- to 6-membered

heterocycloalkyl is optionally substituted with one or more $R^A$;

$R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;

$G_1$ is selected from the group consisting of -N- and $-CR^{3A}-$;

$G_2$ is selected from the group consisting of -N- and $-CR^{3B}-$;

$R^{3A}$, $R^{3B}$, and $R^3$ are each independently selected from the group consisting of hydrogen, halogen, hydroxy, cyano, $C_{1-6}$ alkyl, $C_{1-6}$ alkoxy, $C_{1-6}$ haloalkyl, $C_{1-6}$ haloalkoxy, 3- to 6-membered cycloalkyl, and 4- to 10-membered heterocycloalkyl, and the heterocycloalkyl comprises 1, 2, or 3 heteroatoms selected from the group consisting of O and N, -CONR'R", or -NH-C(O)-OR'; the $C_{1-6}$ alkyl, $C_{1-6}$ alkoxy, $C_{1-6}$ haloalkyl, $C_{1-6}$ haloalkoxy, 3- to 6-membered cycloalkyl, or 4- to 10-membered heterocycloalkyl is optionally substituted with one or more $R^B$, and $R^B$ is selected from the group consisting of halogen, hydroxy, cyano, $C_{1-6}$ alkyl, $C_{1-6}$ haloalkyl, $C_{1-6}$ alkoxy, and 3- to 6-membered cycloalkyl;

R' and R" are each independently selected from the group consisting of hydrogen, $C_{1-6}$ alkyl, and $C_{1-6}$ haloalkyl;

ring B is selected from the group consisting of 5- to 6-membered heterocycloalkyl and 5- to 6-membered heteroaryl, and the 5- to 6-membered heterocycloalkyl or 5- to 6-membered heteroaryl optionally comprises at least one nitrogen atom and/or at least one oxo group;

each $R^4$ is independently selected from the group consisting of halogen, hydroxy, cyano, $C_{1-6}$ alkyl, $C_{1-6}$ alkoxy, $C_{1-6}$ haloalkyl, $C_{1-6}$ haloalkoxy, 3- to 6-membered cycloalkyl, and 4- to 10-membered heterocycloalkyl, and the heterocycloalkyl comprises 1, 2, or 3 heteroatoms selected from the group consisting of O and N; the $C_{1-6}$ alkyl, $C_{1-6}$ alkoxy, $C_{1-6}$ haloalkyl, $C_{1-6}$ haloalkoxy, 3- to 6-membered cycloalkyl, or 4- to 10-membered heterocycloalkyl is optionally substituted with one or more $R^C$, and $R^C$ is selected from the group consisting of halogen, hydroxy, cyano, $C_{1-6}$ alkyl, $C_{1-6}$ haloalkyl, $C_{1-6}$ alkoxy, and 3- to 6-membered cycloalkyl;

m is selected from the group consisting of 0, 1, 2, and 3;

n is selected from the group consisting of 0, 1, 2, and 3; and

s is selected from the group consisting of 0, 1, 2, and 3.

In some embodiments, provided in the present disclosure is the compound represented by formula (I) or the pharmaceutically acceptable salt thereof, wherein ring A is 3- to 7-membered cycloalkyl, e.g., cyclopropyl, cyclobutyl, cyclopentyl, cyclohexyl, or cycloheptyl.

In some embodiments, ring A is cyclopentyl.

In some embodiments, provided in the present disclosure is the compound represented by formula (I) or the pharmaceutically acceptable salt thereof, wherein ring A is 3- to 7-membered heterocycloalkyl (e.g., 3-membered, 4-membered, 5-membered, 6-membered, or 7-membered), and the heterocycloalkyl comprises at least one heteroatom selected from the group consisting of O, N, and S.

In an optional embodiment, ring A is 4- to 6-membered heterocycloalkyl, and the heterocycloalkyl comprises one or two heteroatoms selected from the group consisting of O, N, and S.

In an optional embodiment, ring A is 5- to 6-membered heterocycloalkyl, and the heterocycloalkyl comprises one or two heteroatoms selected from the group consisting of O, N, and S.

In an optional embodiment, ring A is 5- to 6-membered heterocycloalkyl, and the heterocycloalkyl comprises one or two O atoms.

In an optional embodiment, ring A is 5- to 6-membered heterocycloalkyl, and the heterocycloalkyl comprises one O atom.

In an optional embodiment, ring A is 5- to 6-membered heterocycloalkyl, and the heterocycloalkyl comprises two O atoms.

In some embodiments, the compound represented by formula (I) or the pharmaceutically acceptable salt thereof provided in the present disclosure is a compound represented by formula (I-1) or a pharmaceutically acceptable salt thereof,

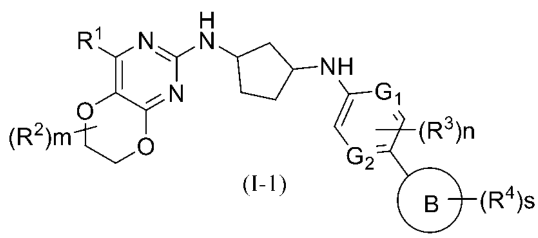

(I-1)

wherein ring B, $R^1$, $R^2$, $G_1$, $G_2$, $R^3$, $R^4$, m, n, and s are as defined in the compound represented by formula (I) or the

pharmaceutically acceptable salt thereof.

In some embodiments, the compound represented by formula (I) or the pharmaceutically acceptable salt thereof provided in the present disclosure is a compound represented by formula (I-2) or a pharmaceutically acceptable salt thereof,

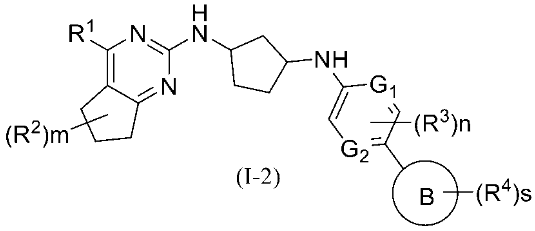

(I-2)

wherein ring B, $R^1$, $R^2$, $G_1$, $G_2$, $R^3$, $R^4$, m, n, and s are as defined in the compound represented by formula (I) or the pharmaceutically acceptable salt thereof.

In an optional embodiment, the compound of (I) or the pharmaceutically acceptable salt thereof provided in the present disclosure is a compound represented by formula (I-3) or a pharmaceutically acceptable salt thereof,

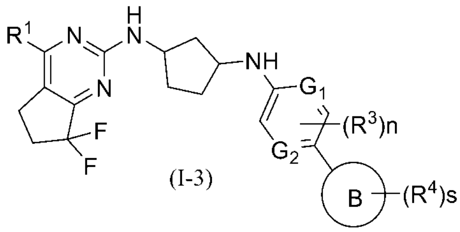

(I-3)

wherein ring B, $R^1$, $G_1$, $G_2$, $R^3$, $R^4$, n, and s are as defined in the compound represented by formula (I) or the pharmaceutically acceptable salt thereof.

In an optional embodiment, the compound of (I) or the pharmaceutically acceptable salt thereof provided in the present disclosure is a compound represented by formula (I-4) or a pharmaceutically acceptable salt thereof,

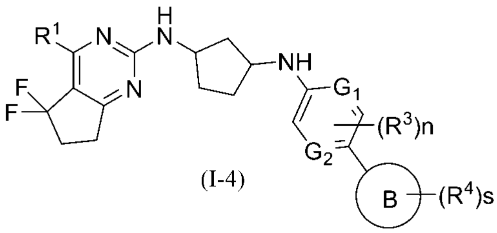

(I-4)

wherein ring B, $R^1$, $G_1$, $G_2$, $R^3$, $R^4$, n, and s are as defined in the compound represented by formula (I) or the pharmaceutically acceptable salt thereof.

In an optional embodiment, the compound of (I) or the pharmaceutically acceptable salt thereof provided in the present disclosure is a compound represented by formula (I-5) or a pharmaceutically acceptable salt thereof,

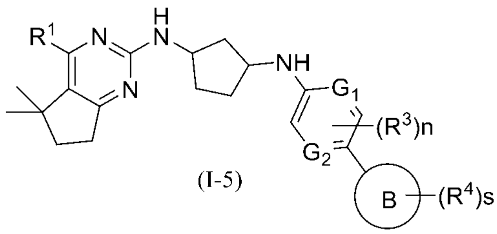

(I-5)

wherein ring B, $R^1$, $G_1$, $G_2$, $R^3$, $R^4$, n, and s are as defined in the compound represented by formula (I) or the pharmaceutically acceptable salt thereof.

In an optional embodiment, the compound of (I) or the pharmaceutically acceptable salt thereof provided in the present disclosure is a compound represented by formula (I-6) or a pharmaceutically acceptable salt thereof,

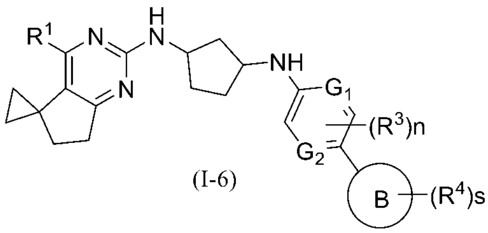

(I-6)

wherein ring B, $R^1$, $G_1$, $G_2$, $R^3$, $R^4$, n, and s are as defined in the compound represented by formula (I) or the pharmaceutically acceptable salt thereof.

In some embodiments, provided in the present disclosure is the compound represented by formula (I), (I-1), (I-2), (I-3), (I-4), (I-5), or (I-6) or the pharmaceutically acceptable salt thereof, wherein $G_1$ is -N-, and $G_2$ is $-CR^{3B}-$.

In some embodiments, provided in the present disclosure is the compound represented by formula (I), (I-1), (I-2), (I-3), (I-4), (I-5), or (I-6) or the pharmaceutically acceptable salt thereof, wherein $G_1$ is -N-, and $G_2$ is -N-.

In some embodiments, provided in the present disclosure is the compound represented by formula (I), (I-1), (I-2), (I-3), (I-4), (I-5), or (I-6) or the pharmaceutically acceptable salt thereof, wherein $G_1$ is $-CR^{3A}-$, and $G_2$ is $-CR^{3B}-$.

In some embodiments, provided in the present disclosure is the compound represented by formula (I), (I-1), (I-2), (I-3), (I-4), (I-5), or (I-6) or the pharmaceutically acceptable salt thereof, wherein ring B is selected from the group consisting of

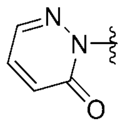 ,

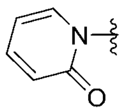 , 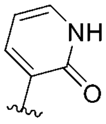 , 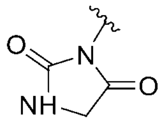 , 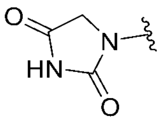 , 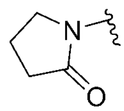 , 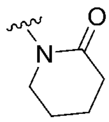 , and 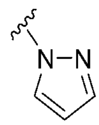 .

In an optional embodiment, provided in the present disclosure is the compound represented by formula (I), (I-1), (I-2), (I-3), (I-4), (I-5), or (I-6) or the pharmaceutically acceptable salt thereof, wherein ring B is

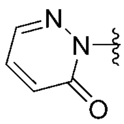 .

In an optional embodiment, provided in the present disclosure is the compound represented by formula (I), (I-1), (I-2), (I-3), (I-4), (I-5), or (I-6) or the pharmaceutically acceptable salt thereof, wherein ring B is

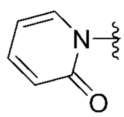 .

In an optional embodiment, provided in the present disclosure is the compound represented by formula (I), (I-1), (I-2), (I-3), (I-4), (I-5), or (I-6) or the pharmaceutically acceptable salt thereof, wherein ring B is

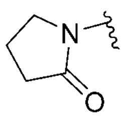 .

In an optional embodiment, provided in the present disclosure is the compound represented by formula (I), (I-1), (I-2), (I-3), (I-4), (I-5), or (I-6) or the pharmaceutically acceptable salt thereof, wherein ring B is

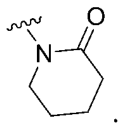

In an optional embodiment, provided in the present disclosure is the compound represented by formula (I), (I-1), (I-2), (I-3), (I-4), (I-5), or (I-6) or the pharmaceutically acceptable salt thereof, wherein ring B is

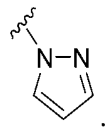

In some embodiments, provided in the present disclosure is the compound represented by formula (I), (I-1), (I-2), (I-3), (I-4), (I-5), or (I-6) or the pharmaceutically acceptable salt thereof, wherein each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano.

In some embodiments, provided in the present disclosure is the compound represented by formula (I), (I-1), (I-2), (I-3), (I-4), (I-5), or (I-6) or the pharmaceutically acceptable salt thereof, wherein each $R^4$ is independently selected from the group consisting of fluorine, chlorine, cyano, methyl, trifluoromethyl, trifluoromethoxy, cyclopropyl, and cyanomethyl.

In some embodiments, provided in the present disclosure is the compound represented by formula (I), (I-1), (I-2), (I-3), (I-4), (I-5), or (I-6) or the pharmaceutically acceptable salt thereof, wherein $R^{3A}$, $R^{3B}$, and $R^3$ are each independently selected from the group consisting of hydrogen, halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano.

In some embodiments, provided in the present disclosure is the compound represented by formula (I), (I-1), (I-2), (I-3), (I-4), (I-5), or (I-6) or the pharmaceutically acceptable salt thereof, wherein $R^{3A}$, $R^{3B}$, and $R^3$ are each independently selected from the group consisting of hydrogen, fluorine, chlorine, cyano, methyl, trifluoromethyl, trifluoromethoxy, cyclopropyl, and cyanomethyl.

In some embodiments, the compound represented by formula (I) or the pharmaceutically acceptable salt thereof provided in the present disclosure is a compound represented by formula (II-A-1) or a pharmaceutically acceptable salt thereof,

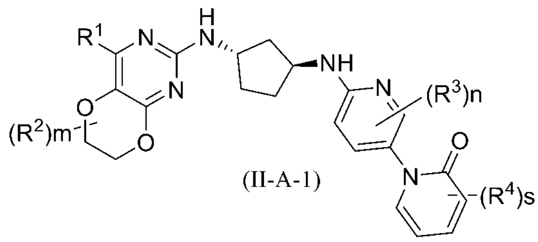

(II-A-1)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-A-2) or a pharmaceutically acceptable salt thereof,

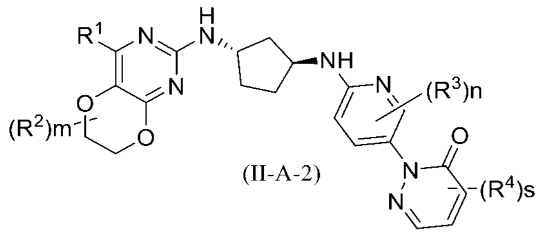

(II-A-2)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-A-3) or a pharmaceutically acceptable salt thereof,

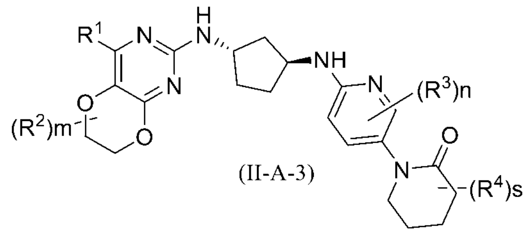

(II-A-3)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-A-4) or a pharmaceutically acceptable salt thereof,

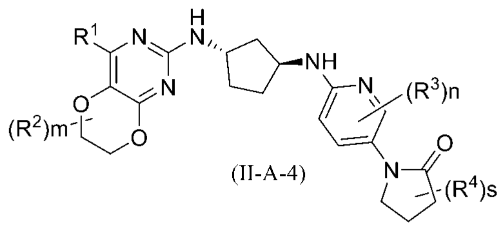

(II-A-4)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-A-5) or a pharmaceutically acceptable salt thereof:

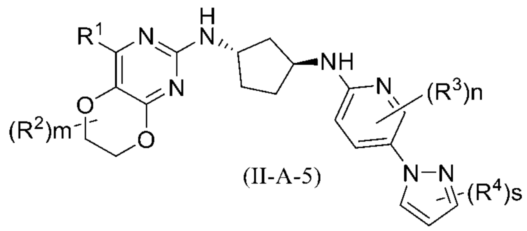

(II-A-5)

,

wherein $R^1$, $R^2$, $R^3$, $R^4$, m, n, and s are as defined in the compound represented by formula (I) or the pharmaceutically acceptable salt thereof.

In some embodiments, provided in the present disclosure is the compound represented by formula (II-A-1), (II-A-2), (II-A-3), (II-A-4), or (II-A-5) or the pharmaceutically acceptable salt thereof, wherein:

$R^1$ is hydrogen;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$, and $R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl,

$C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;

m, n, and s are each independently selected from the group consisting of 0, 1, 2, and 3.

In some embodiments, provided in the present disclosure is the compound represented by formula (II-A-1), (II-A-2), (II-A-3), (II-A-4), or (II-A-5) or the pharmaceutically acceptable salt thereof, wherein:

$R^1$ is hydrogen;

each $R^3$ is independently selected from the group consisting of fluorine, chlorine, cyano, methyl, trifluoromethyl, and trifluoromethoxy;

each $R^4$ is independently selected from the group consisting of fluorine, chlorine, cyano, methyl, trifluoromethyl, trifluoromethoxy, cyclopropyl, and cyanomethyl;

m is 0;

n and s are each independently selected from the group consisting of 0, 1, and 2.

In some embodiments, the compound represented by formula (I) or the pharmaceutically acceptable salt thereof provided in the present disclosure is a compound represented by formula (II-B-1) or a pharmaceutically acceptable salt thereof,

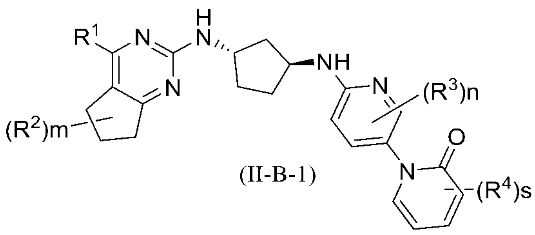

(II-B-1)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-B-2) or a pharmaceutically acceptable salt thereof,

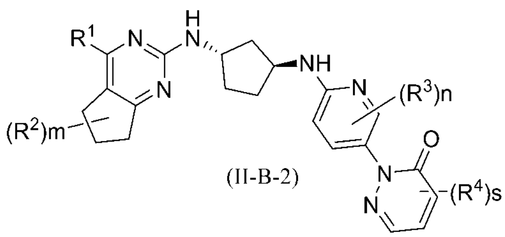

(II-B-2)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-B-3) or a pharmaceutically acceptable salt thereof,

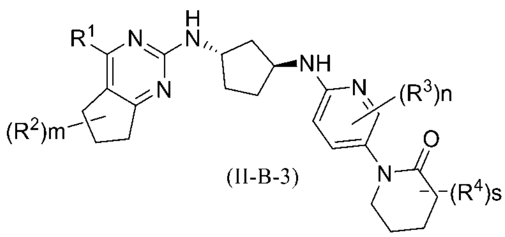

(II-B-3)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-B-4) or a pharmaceutically acceptable salt thereof,

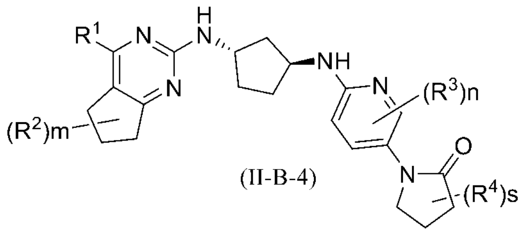

(II-B-4)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-B-5) or a pharmaceutically acceptable salt thereof,

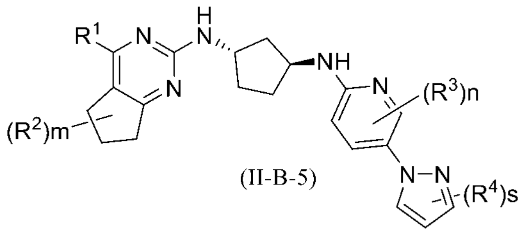

(II-B-5)

wherein $R^1$, $R^2$, $R^3$, $R^4$, m, n, and s are as defined in the compound represented by formula (I) or the pharmaceutically acceptable salt thereof.

In an optional embodiment, provided in the present disclosure is the compound represented by formula (II-B-1), (II-B-2), (II-B-3), (II-B-4), or (II-B-5) or the pharmaceutically acceptable salt thereof, wherein:

$R^1$ is hydrogen;
$R^2$ is selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$, and $R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3.

In an optional embodiment, provided in the present disclosure is the compound represented by formula (II-B-1), (II-B-2), (II-B-3), (II-B-4), or (II-B-5) or the pharmaceutically acceptable salt thereof, wherein:

$R^1$ is hydrogen;
two $R^2$ attached to the same carbon atom, together with the carbon atom to which they are attached, form 3- to 6-membered cycloalkyl, wherein the 3- to 6-membered cycloalkyl is optionally substituted with one or more $R^A$, and $R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl,

$C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;

each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;

m, n, and s are each independently selected from the group consisting of 0, 1, 2, and 3.

In an optional embodiment, provided in the present disclosure is the compound represented by formula (II-B-1), (II-B-2), (II-B-3), (II-B-4), or (II-B-5) or the pharmaceutically acceptable salt thereof, wherein:

$R^1$ is hydrogen;

each $R^2$ is independently selected from the group consisting of fluorine and methyl;

each $R^3$ is independently selected from the group consisting of fluorine, chlorine, cyano, methyl, trifluoromethyl, and trifluoromethoxy;

each $R^4$ is independently selected from the group consisting of fluorine, chlorine, cyano, methyl, trifluoromethyl, trifluoromethoxy, cyclopropyl, and cyanomethyl;

m, n, and s are each independently selected from the group consisting of 0, 1, 2, and 3.

In an optional embodiment, provided in the present disclosure is the compound represented by formula (II-B-1), (II-B-2), (II-B-3), (II-B-4), or (II-B-5) or the pharmaceutically acceptable salt thereof, wherein:

$R^1$ is hydrogen;

two $R^2$ attached to the same carbon atom, together with the carbon atom to which they are attached, form cyclopropyl, wherein the cyclopropyl is optionally substituted with one or more $R^A$, and $R^A$ is selected from the group consisting of halogen;

each $R^3$ is independently selected from the group consisting of fluorine, chlorine, cyano, methyl, trifluoromethyl, and trifluoromethoxy;

each $R^4$ is independently selected from the group consisting of fluorine, chlorine, cyano, methyl, trifluoromethyl, trifluoromethoxy, cyclopropyl, and cyanomethyl;

m, n, and s are each independently selected from the group consisting of 0, 1, 2, and 3.

In some embodiments, the compound represented by formula (I) or the pharmaceutically acceptable salt thereof provided in the present disclosure is a compound represented by formula (II-C-1) or a pharmaceutically acceptable salt thereof,

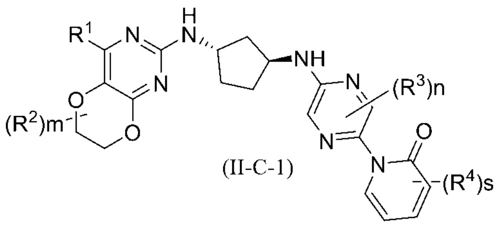

(II-C-1)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-C-2) or a pharmaceutically acceptable salt thereof,

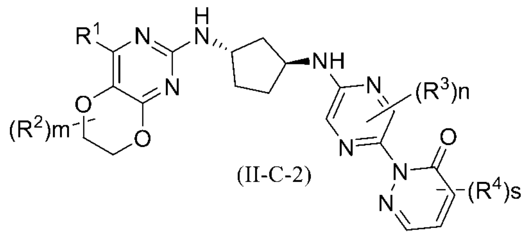

(II-C-2)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-C-3) or a pharmaceutically acceptable salt thereof,

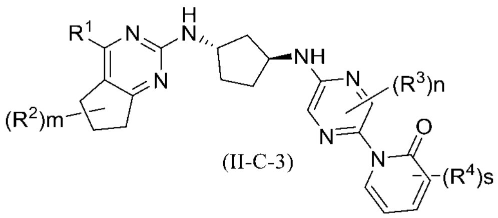

(II-C-3)

wherein $R^1$, $R^2$, $R^3$, $R^4$, m, n, and s are as defined in the compound represented by formula (I) or the pharmaceutically acceptable salt thereof.

In some embodiments, the compound represented by formula (I) or the pharmaceutically acceptable salt thereof provided in the present disclosure is a compound represented by formula (II-C-4) or a pharmaceutically acceptable salt thereof,

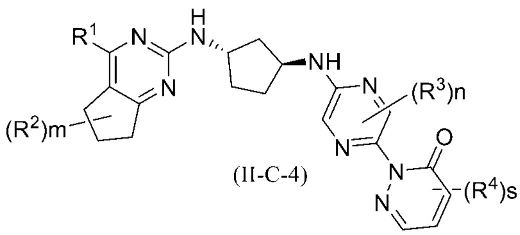

(II-C-4)

wherein $R^1$, $R^2$, $R^3$, $R^4$, m, n, and s are as defined in the compound represented by formula (I) or the pharmaceutically acceptable salt thereof.

In an optional embodiment, provided in the present disclosure is the compound represented by formula (II-C-1) or (II-C-2) or the pharmaceutically acceptable salt thereof, wherein:

$R^1$ is hydrogen;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, and the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$;
$R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3.

In an optional embodiment, provided in the present disclosure is the compound represented by formula (II-C-1) or (II-C-2) or the pharmaceutically acceptable salt thereof, wherein:

$R^1$ is hydrogen;
each $R^3$ is independently selected from the group consisting of fluorine, chlorine, cyano, methyl, trifluoromethyl, and trifluoromethoxy;
each $R^4$ is independently selected from the group consisting of fluorine, chlorine, cyano, methyl, trifluoromethyl, trifluoromethoxy, cyclopropyl, and cyanomethyl;
m is 0;
n and s are each independently selected from the group consisting of 0, 1, 2, and 3.

In an optional embodiment, provided in the present disclosure is the compound represented by formula (II-C-3) or (II-C-4) or the pharmaceutically acceptable salt thereof, wherein:

$R^1$ is hydrogen;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$, and $R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3.

In an optional embodiment, provided in the present disclosure is the compound represented by formula (II-C-3) or (II-C-4) or the pharmaceutically acceptable salt thereof, wherein:

$R^1$ is hydrogen;
two $R^2$ attached to the same carbon atom, together with the carbon atom to which they are attached, form 3- to 6-membered cycloalkyl, wherein the 3- to 6-membered cycloalkyl is optionally substituted with one or more $R^A$, and $R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3.

In an optional embodiment, provided in the present disclosure is the compound represented by formula (II-C-3) or (II-C-4) or the pharmaceutically acceptable salt thereof, wherein:

$R^1$ is hydrogen;
each $R^2$ is independently selected from the group consisting of fluorine and methyl;
each $R^3$ is independently selected from the group consisting of fluorine, chlorine, cyano, methyl, trifluoromethyl, and trifluoromethoxy;
each $R^4$ is independently selected from the group consisting of fluorine, chlorine, cyano, methyl, trifluoromethyl, trifluoromethoxy, cyclopropyl, and cyanomethyl;
m, n, and s are each independently selected from the group consisting of 0, 1, 2, and 3.

In an optional embodiment, provided in the present disclosure is the compound represented by formula (II-C-3) or (II-C-4) or the pharmaceutically acceptable salt thereof, wherein:

$R^1$ is hydrogen;
two $R^2$ attached to the same carbon atom, together with the carbon atom to which they are attached, form cyclopropyl, wherein the cyclopropyl is optionally substituted with one or more $R^A$, and $R^A$ is a halogen;
each $R^3$ is independently selected from the group consisting of fluorine, chlorine, cyano, methyl, trifluoromethyl, and trifluoromethoxy;
each $R^4$ is independently selected from the group consisting of fluorine, chlorine, cyano, methyl, trifluoromethyl, trifluoromethoxy, cyclopropyl, and cyanomethyl;
m, n, and s are each independently selected from the group consisting of 0, 1, 2, and 3.

The present disclosure further provides the following compounds or pharmaceutically acceptable salts thereof:

group A

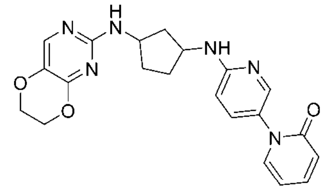,
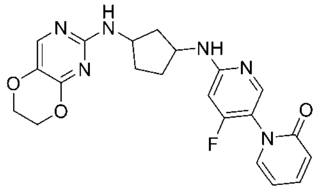,
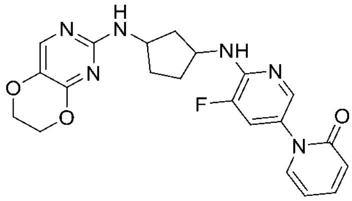,
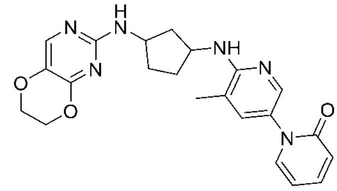,
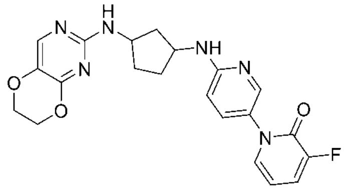,
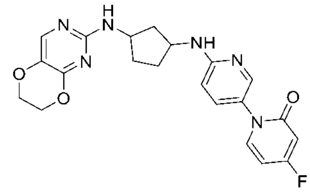,
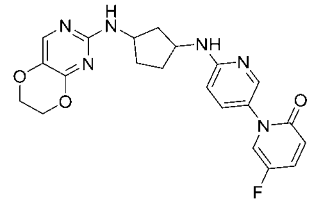,
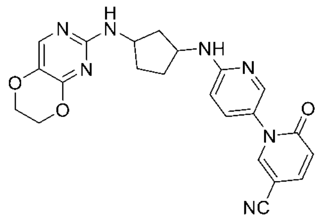,
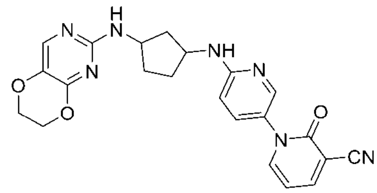,
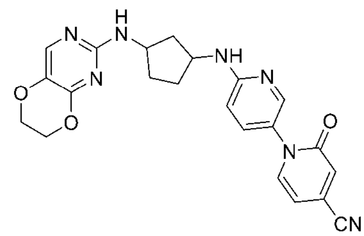,
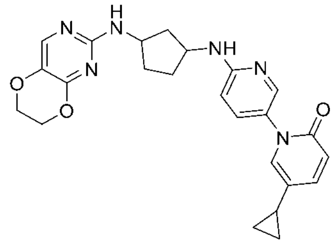,
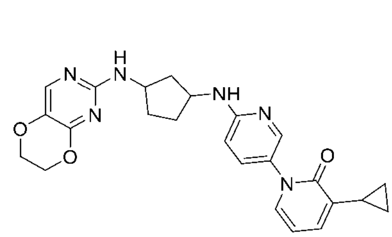,
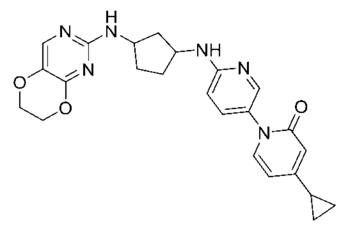;

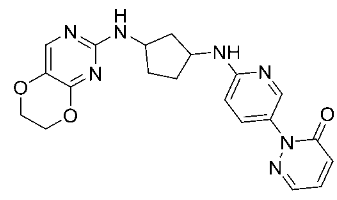,
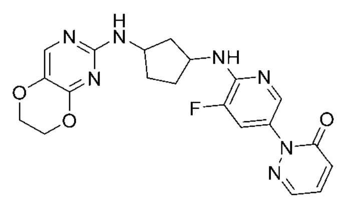,
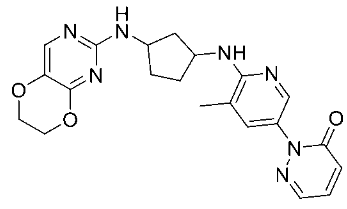,
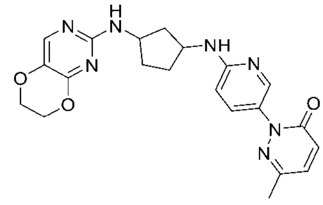,
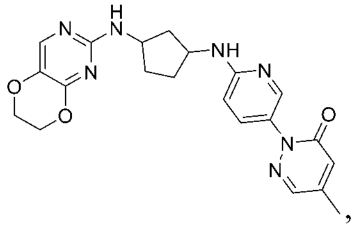,
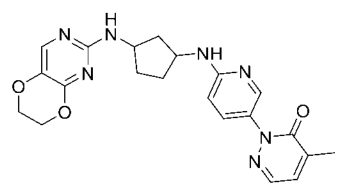;

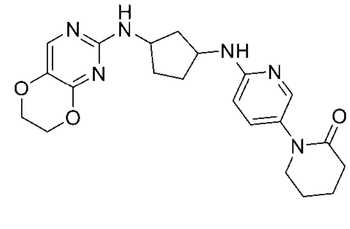,
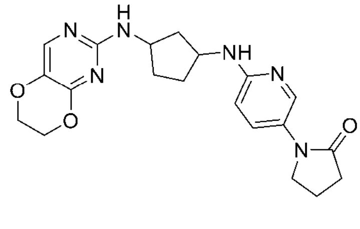,
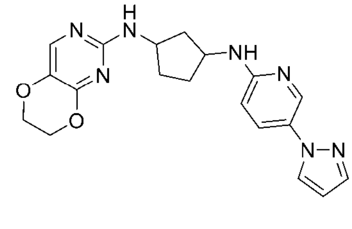;

group B

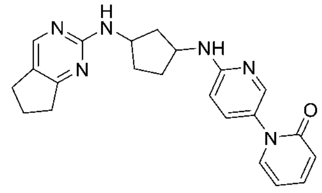, 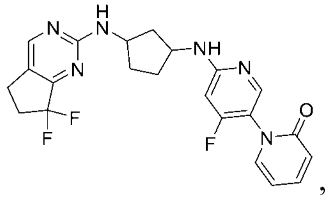, 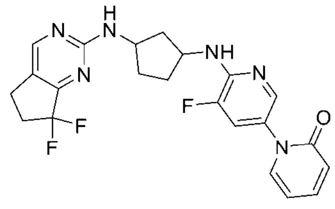,

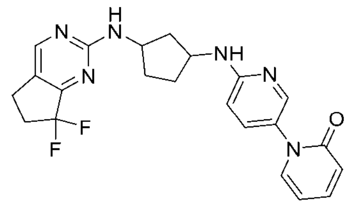, 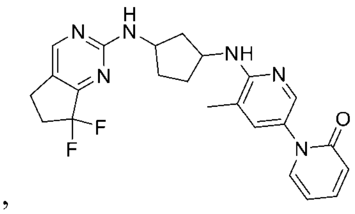, 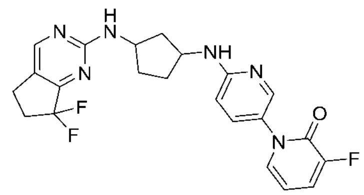,

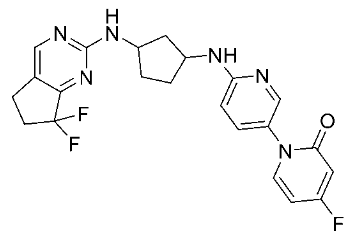, 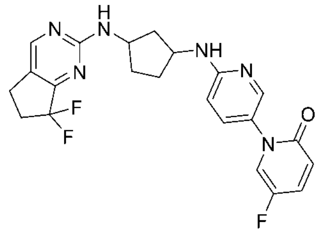, 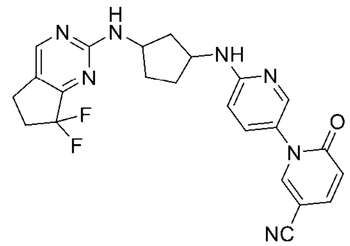,

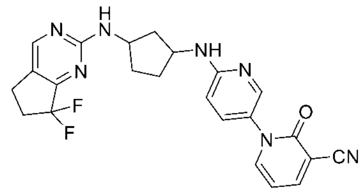, 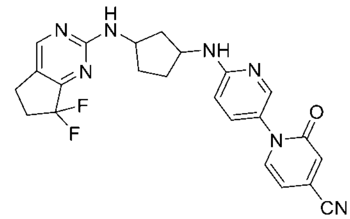, 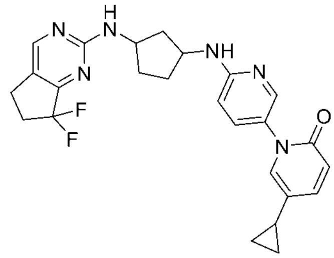,

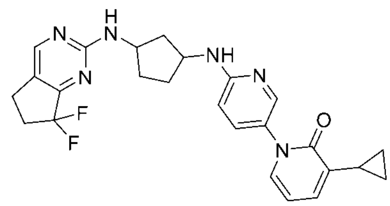, 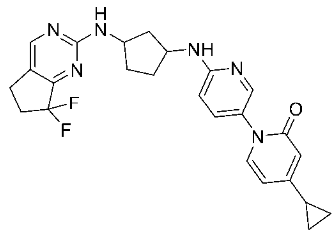, 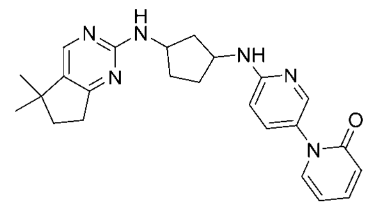,

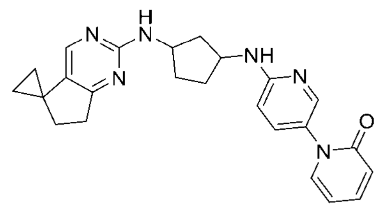, 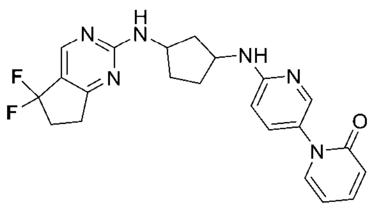;

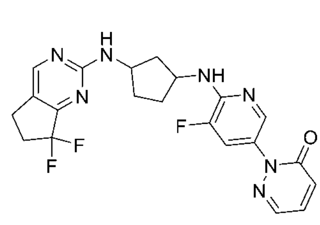, 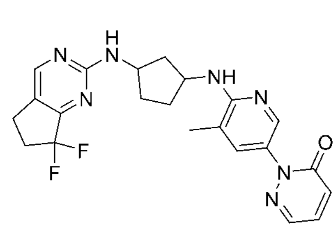, 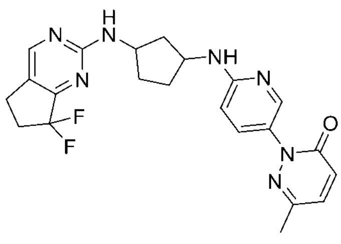,

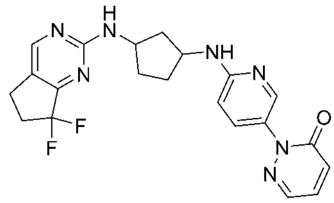,
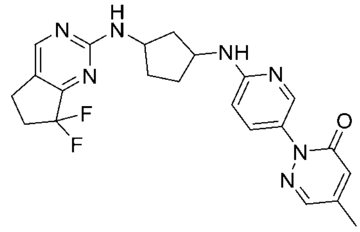,
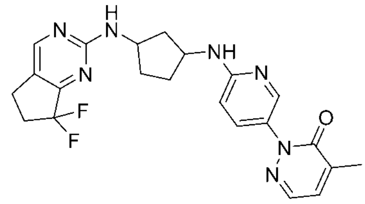,
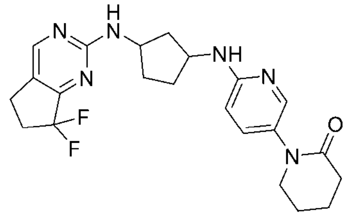,
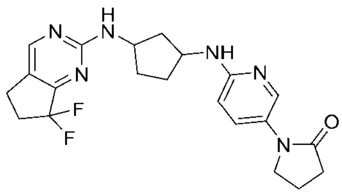,
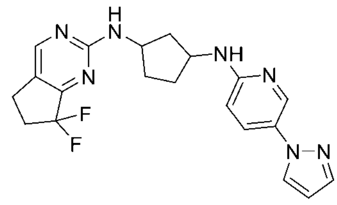;
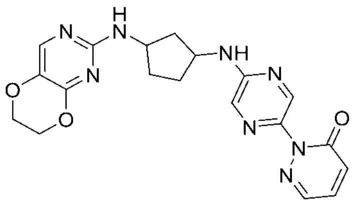,
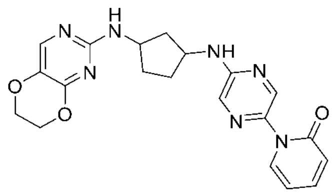,
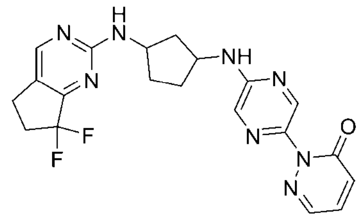,
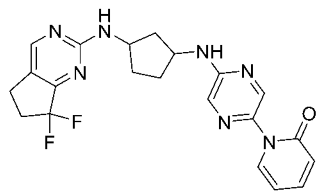,
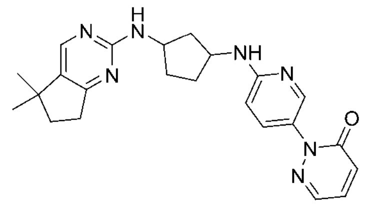,
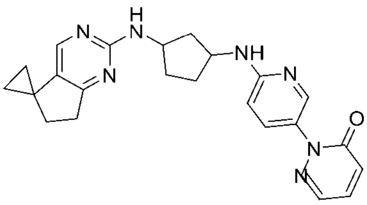,
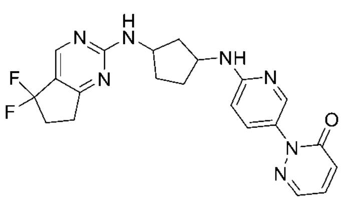,
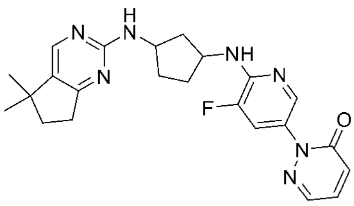,
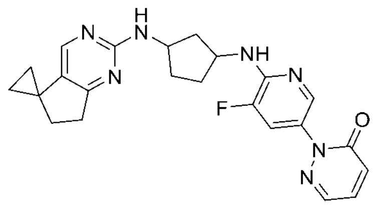,
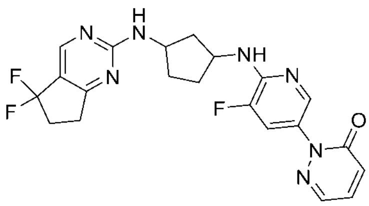;

group C

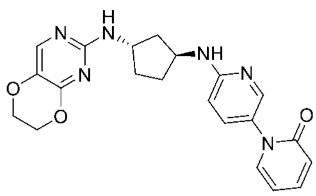, 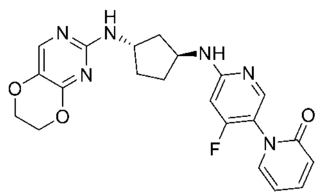, 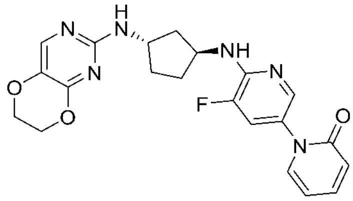,

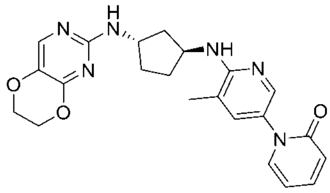, 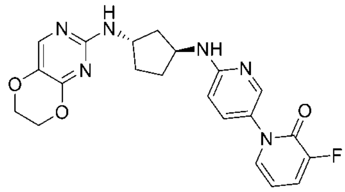, 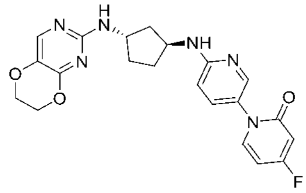,

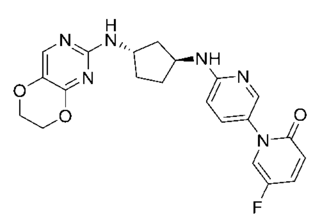, 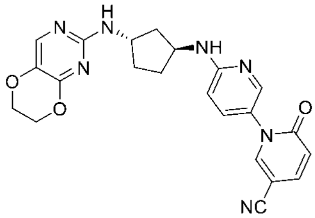, 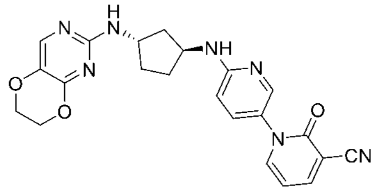,

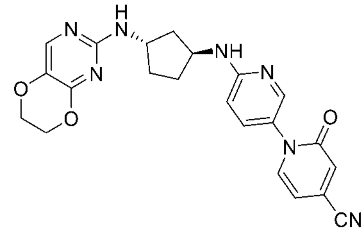, 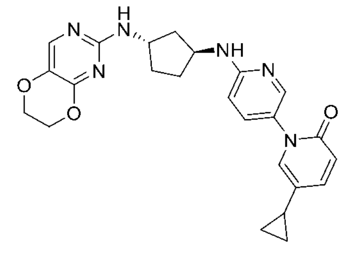, 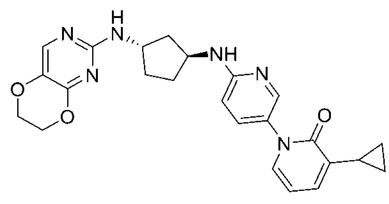,

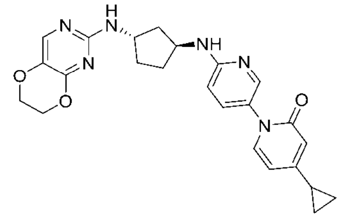;

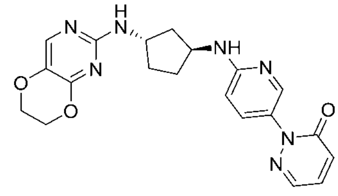, 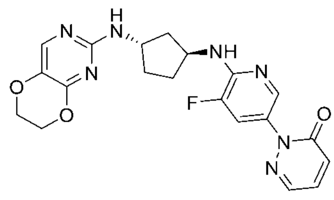, 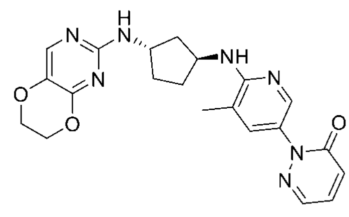,

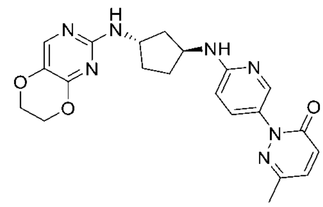, 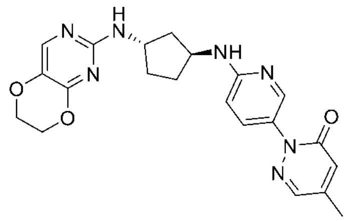, 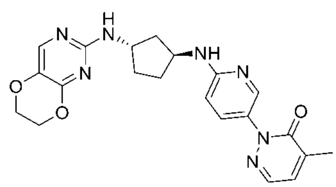;

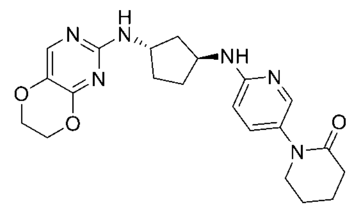, 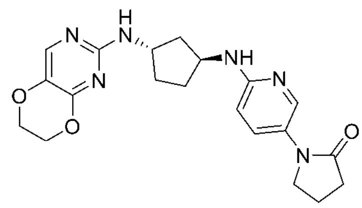, 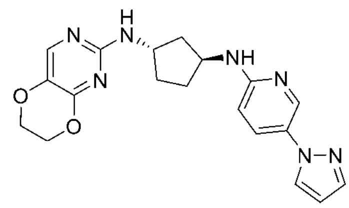;

group D

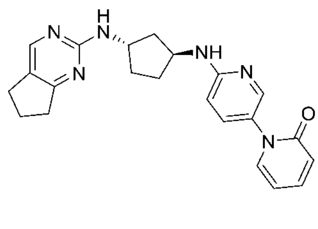, 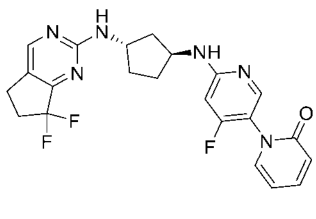, 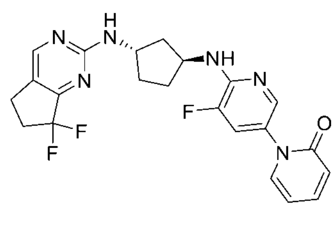,

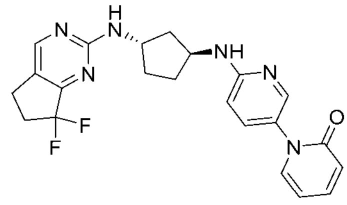,
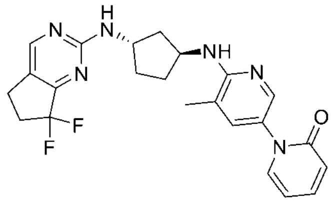,
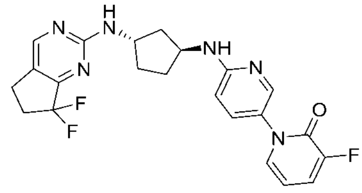,
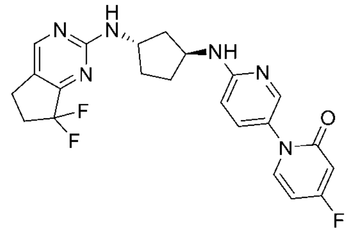,
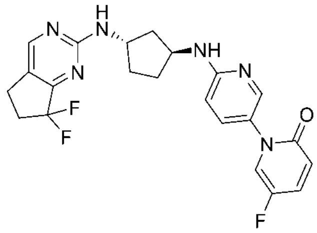,
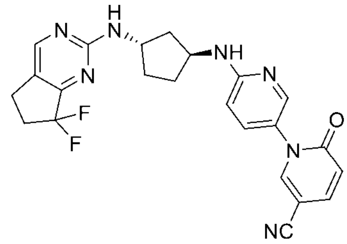,
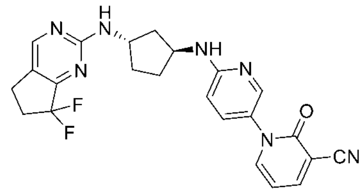,
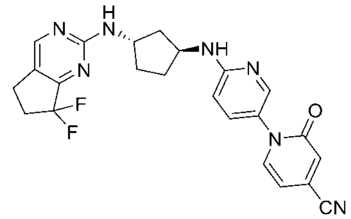,
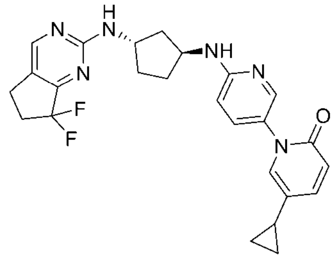,
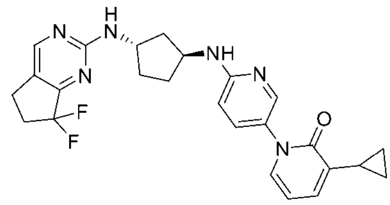,
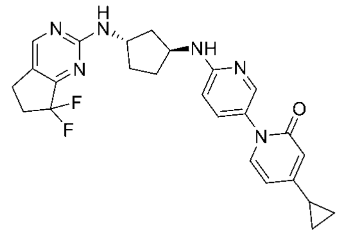,
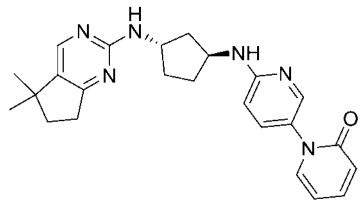,
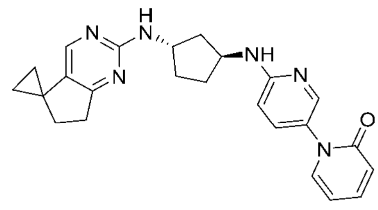,
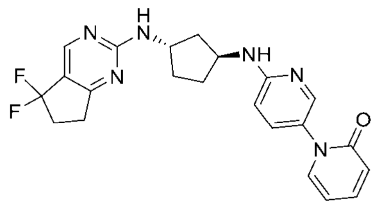;
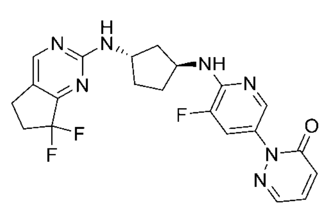,
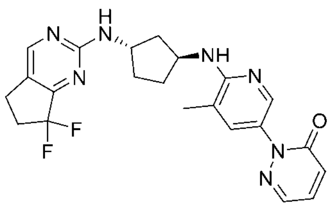,
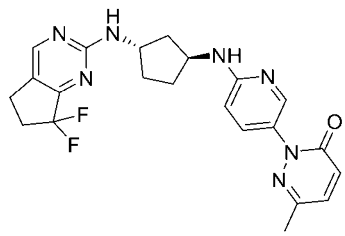,
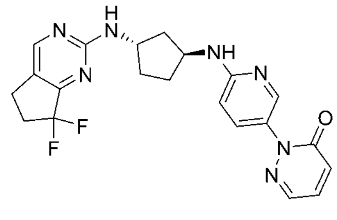,
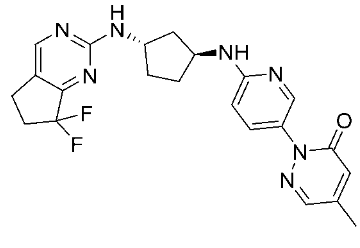,
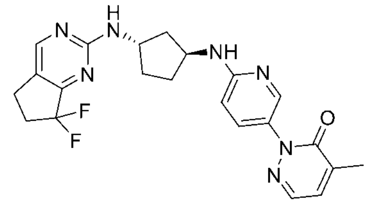,

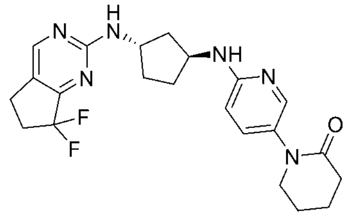, 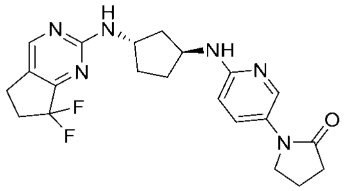, 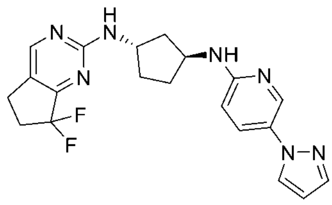;

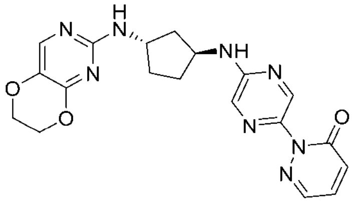, 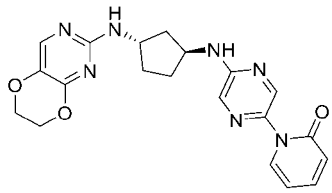, 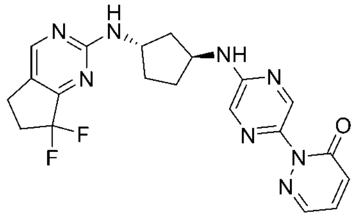,

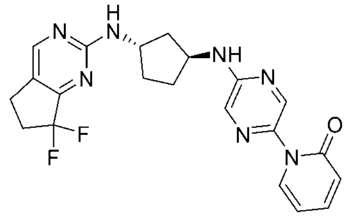, 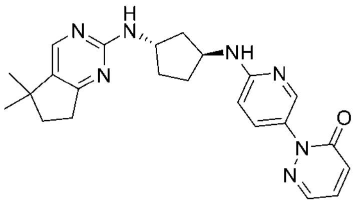, 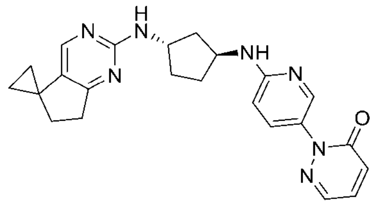,

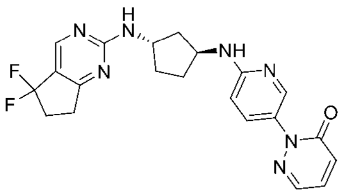, 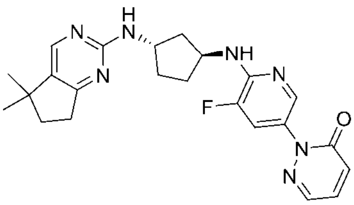, 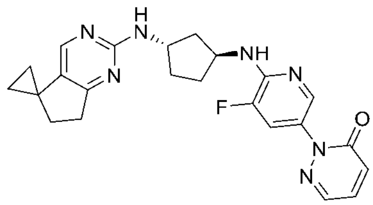,

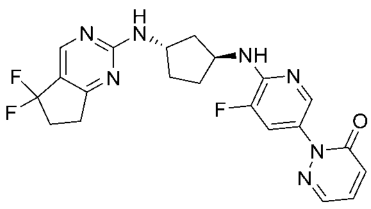, and 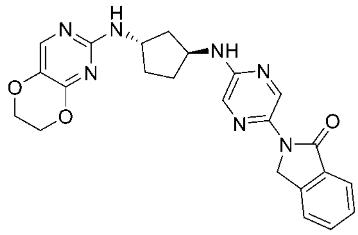.

Another aspect of the present disclosure provides an isotopically substituted form of the aforementioned compound or the pharmaceutically acceptable salt thereof, wherein the isotopically substituted form is a deuterated form.

The present disclosure further provides a pharmaceutical composition comprising the aforementioned compound or the pharmaceutically acceptable salt thereof or the isotopically substituted form and at least one pharmaceutically acceptable excipient.

In an optional embodiment, the pharmaceutical composition comprises an effective amount of the aforementioned compound or the pharmaceutically acceptable salt thereof or the isotopically substituted form.

Another aspect of the present disclosure provides the aforementioned compound or the pharmaceutically acceptable salt thereof or the isotopically substituted form for use as a medicament.

Another aspect of the present disclosure provides use of the aforementioned compound or the pharmaceutically acceptable salt thereof, the isotopically substituted form, or the composition in the manufacture of a medicament of a PCSK9 inhibitor.

Another aspect of the present disclosure provides use of the aforementioned compound or the pharmaceutically acceptable salt thereof, the isotopically substituted form, or the composition in the manufacture of a medicament for preventing and/or treating a disease and a disorder.

The present disclosure further provides a method for preventing and/or treating a disease and a disorder, comprising administering the aforementioned compound or the pharmaceutically acceptable salt thereof, the isotopically substituted form, or the composition.

The present disclosure further provides the aforementioned compound or the pharmaceutically acceptable salt thereof, the isotopically substituted form, or the composition for use in preventing and/or treating a disease and a disorder.

The disease and the disorder described in the present disclosure are selected from the group consisting of dyslipidemia, dyslipoproteinemia, hypercholesterolemia, hyperlipidemia, hypertriglyceridemia, hyperlipoproteinemia, xanthoma, hypoalphalipoproteinemia, sitosterolemia, atherosclerosis, arteriosclerosis, metabolic syndrome, coronary heart disease, peripheral vascular disease, congestive heart failure, stroke, vascular dementia, coronary artery disease, chronic kidney disease, retinopathy, inflammation, diabetic complications, and thrombi.

In certain embodiments, the disease and the disorder are selected from the group consisting of hypercholesterolemia, hyperlipidemia, hyperlipoproteinemia, hypertriglyceridemia, dyslipidemia, dyslipoproteinemia, atherosclerosis, hepatic steatosis, metabolic syndrome, and coronary artery disease.

In certain embodiments, the disease is hypercholesterolemia, such as familial hypercholesterolemia or autosomal dominant hypercholesterolemia.

In certain embodiments, the disease is hyperlipidemia.

In certain embodiments, the disease is coronary artery disease.

Another aspect of the present disclosure provides use of the aforementioned compound or the pharmaceutically acceptable salt thereof, the isotopically substituted form, or the composition in the manufacture of a medicament for reducing lipoprotein(a) (LP(a)), low-density lipoprotein (LDL), very low-density lipoprotein (VLDL), triglyceride-rich lipoprotein (TRL), or triglyceride.

Another aspect of the present disclosure provides use of the aforementioned compound or the pharmaceutically acceptable salt thereof, the isotopically substituted form, or the composition in the manufacture of a medicament for preventing and/or treating a PCSK9-mediated disease and a PCSK9-mediated disorder.

The present disclosure further provides a method for preventing and/or treating a PCSK9-mediated disease and a PCSK9-mediated disorder, comprising administering the aforementioned compound or the pharmaceutically acceptable salt thereof, the isotopically substituted form, or the composition.

The present disclosure further provides the aforementioned compound or the pharmaceutically acceptable salt thereof, the isotopically substituted form, or the composition for use in preventing and/or treating a PCSK9-mediated disease and a PCSK9-mediated disorder.

The PCSK9-mediated disease and the PCSK9-mediated disorder described in the present disclosure are selected from the group consisting of dyslipidemia, dyslipoproteinemia, hypercholesterolemia, hyperlipidemia, hypertriglyceridemia, hyperlipoproteinemia, xanthoma, hypoalphalipoproteinemia, sitosterolemia, atherosclerosis, arteriosclerosis, metabolic syndrome, coronary heart disease, peripheral vascular disease, congestive heart failure, stroke, vascular dementia, coronary artery disease, chronic kidney disease, retinopathy, inflammation, diabetic complications, and thrombi.

In certain embodiments, the PCSK9-mediated disease and the PCSK9-mediated disorder are selected from the group consisting of hypercholesterolemia, hyperlipidemia, hyperlipoproteinemia, hypertriglyceridemia, dyslipidemia, dyslipoproteinemia, atherosclerosis, hepatic steatosis, metabolic syndrome, and coronary artery disease.

In certain embodiments, the disorder is hypercholesterolemia, such as familial hypercholesterolemia or autosomal dominant hypercholesterolemia.

In certain embodiments, the disease is hyperlipidemia.

In certain embodiments, the disease is coronary artery disease.

Another aspect of the present disclosure provides a method for reducing lipoprotein(a) (LP(a)), low-density lipoprotein (LDL), very low-density lipoprotein (VLDL), triglyceride-rich lipoprotein (TRL), or triglyceride, comprising administering the aforementioned compound or the pharmaceutically acceptable salt thereof, the isotopically substituted form, or the composition.

Another aspect of the present disclosure provides the aforementioned compound or the pharmaceutically acceptable salt thereof, the isotopically substituted form, or the composition for use in reducing lipoprotein(a) (LP(a)), low-density lipoprotein (LDL), very low-density lipoprotein (VLDL), triglyceride-rich lipoprotein (TRL), or triglyceride.

Subjects having a gain-of-function mutation in the PCSK9 gene also benefit from treatment with the disclosed compound and the composition, which counteracts the mutation by inhibiting PCSK9.

In some embodiments, in the method for preventing and/or treating a disease and a disorder or the method for preventing and/or treating a PCSK9-mediated disease and a PCSK9-mediated disorder provided in the present disclosure, the administration subject is a human.

In an optional embodiment, the administration subject is a patient.

In an optional embodiment, the administration subject is a healthy subject.

In an optional embodiment, the administered amount of the aforementioned compound or the pharmaceutically acceptable salt thereof, the isotopically substituted form, or the composition is an effective amount.

In some embodiments, a unit dose of the pharmaceutical composition is 0.001 mg-1000 mg.

In certain embodiments, based on the total weight of the composition, the pharmaceutical composition comprises 0.01-99.99% of the aforementioned compound or the pharmaceutically acceptable salt thereof or the isotopically substituted form thereof. In certain embodiments, the pharmaceutical composition comprises 0.1-99.9%

of the aforementioned compound or the pharmaceutically acceptable salt thereof or the isotopically substituted form thereof. In certain embodiments, the pharmaceutical composition comprises 0.5%-99.5% of the aforementioned compound or the pharmaceutically acceptable salt thereof or the isotopically substituted form thereof. In certain embodiments, the pharmaceutical composition comprises 1%-99% of the aforementioned compound or the pharmaceutically acceptable salt thereof or the isotopically substituted form thereof. In certain embodiments, the pharmaceutical composition comprises 2%-98% of the aforementioned compound or the pharmaceutically acceptable salt thereof or the isotopically substituted form thereof.

In certain embodiments, based on the total weight of the composition, the pharmaceutical composition comprises 0.01%-99.99% of the pharmaceutically acceptable excipient. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The pharmaceutically acceptable salt of the compound described in the present disclosure may be selected from the group consisting of inorganic salts and organic salts.

Another aspect of the present disclosure provides a preparation method for the aforementioned compound or the pharmaceutically acceptable salt thereof, comprising a step of reacting a compound represented by formula (I-a) with a compound represented by formula (I-b) under alkaline conditions, optionally in the presence of a catalyst,

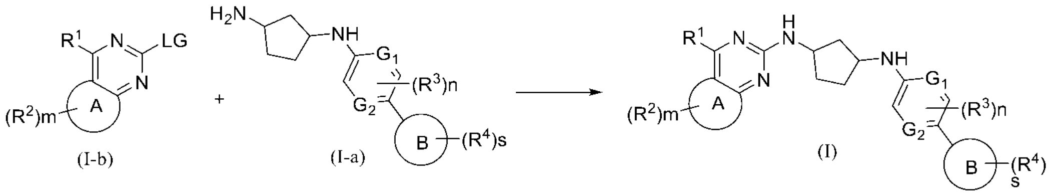

wherein LG is selected from the group consisting of halogen and $-S(O)_2R^7$, and $R^7$ is selected from the group consisting of $C_{1-6}$ alkyl and $C_{1-6}$ haloalkyl; and

$R^1$, $R^2$, $G_1$, $G_2$, $R^3$, $R^4$, ring A, ring B, m, n, and s are as defined in the compound represented by formula (I) or the pharmaceutically acceptable salt thereof.

In some embodiments, LG is selected from the group consisting of halogen, e.g., fluorine or chlorine.

In some embodiments, LG is selected from the group consisting of $-S(O)_2R^7$, and $R^7$ may be selected from the group consisting of methyl, ethyl, etc.

In some embodiments, the alkaline environment is provided by an inorganic or organic base, such as sodium carbonate, potassium carbonate, cesium carbonate, sodium bicarbonate, potassium bicarbonate, pyridine, triethylamine, or N,N-diisopropylethylamine.

In some embodiments, a catalyst suitable for a Buchwald-Hartwig reaction is suitable for the reaction; specifically,

dichloro[1,3-bis(2,6-di-3-pentylphenyl)imidazol-2-ylidene](3-chloropyridyl)palladium(I I) or the like may be selected.

The present disclosure further provides a compound represented by formula (I-b),

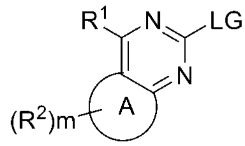

(I-b)

wherein:

LG is selected from the group consisting of halogen and $-S(O)_2R^7$, and $R^7$ is selected from the group consisting of $C_{1-6}$ alkyl and $C_{1-6}$ haloalkyl; $R^1$, $R^2$, and m are as defined in the compound represented by formula (I) or the pharmaceutically acceptable salt thereof.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which fall within the scope of the present disclosure.

Additional asymmetric carbon atoms may be present in substituents such as alkyl. All such isomers and mixtures thereof are included within the scope of the present disclosure. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines). In the chemical structures of the compounds of the present disclosure, the bond "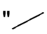" indicates an unspecified configuration; that is, if chiral isomers exist in the chemical structures, the bond "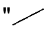" may be "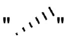" or "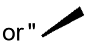", or includes both the configurations "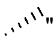" and "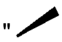" simultaneously. In the chemical structures of the compounds of the present disclosure, the bond "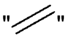" does not specify a configuration; that is, the configuration of the bond "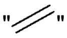" may be an E configuration or a Z configuration, or includes both the E configuration and the Z configuration simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization.

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula (I). Each available hydrogen atom linked to a carbon atom may be independently replaced with a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, etc.

"Optionally" or "optional" means that the event or circumstance subsequently described may, but does not necessarily, occur, and this description includes instances where the event or circumstance occurs or does not occur. For example, "$C_{1-6}$ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

"Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote the dosing of an organism and facilitate the absorption of the active ingredient so that it can exert its biological activity. "Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration (FDA) as acceptable for use in humans or livestock animals.

"Effective amount" or "therapeutically effective amount" as described in the present disclosure includes an

amount sufficient to ameliorate or prevent a symptom or disorder of a medical disorder. An effective amount also means an amount sufficient to allow or facilitate diagnosis. The effective amount for a particular patient or veterinary subject may vary depending on factors such as the disorder to be treated, the general health of the patient, the method, route, and dose of administration, and the severity of side effects. An effective amount may be the maximum dose or administration regimen to avoid significant side effects or toxic effects.

"Alkyl" refers to a saturated aliphatic hydrocarbon group. Non-limiting examples of alkyl groups containing 1 to 6 carbon atoms include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, etc.

The term "cycloalkyl" refers to a saturated or partially unsaturated, monocyclic or polycyclic hydrocarbon substituent, and the cycloalkyl ring contains 3 to 20 carbon atoms, preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc.

The term "heterocycloalkyl" refers to a saturated or partially unsaturated, monocyclic or polycyclic hydrocarbon substituent containing 3 to 20 ring atoms, wherein one or more of the ring atoms are heteroatoms selected from the group consisting of nitrogen, oxygen, and $S(O)_m$ (where m is an integer from 0 to 2), but a ring moiety of -O-O-, -O-S-, or -S-S- is excluded, and the other ring atoms are carbon atoms. Preferably, it contains 3 to 12 ring atoms, 1-4 of which are heteroatoms; more preferably, it contains 4 to 10 ring atoms. Non-limiting examples of "heterocycloalkyl" groups include:

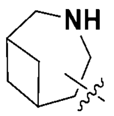, 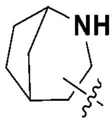, 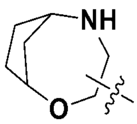, 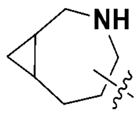, 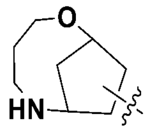, 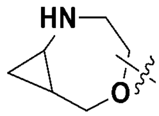,

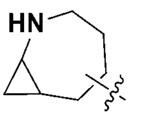, 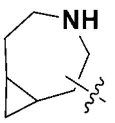, 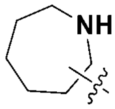, 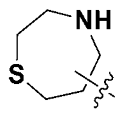, 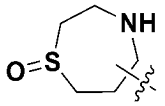, 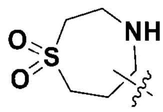,

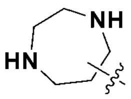, 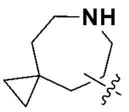, 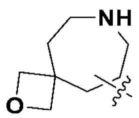, 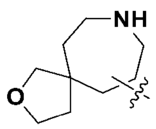, 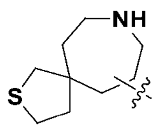 and 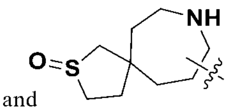.

The heterocycloalkyl ring may be fused to an aryl or heteroaryl ring, wherein the ring attached to the parent structure is heterocycloalkyl; its non-limiting examples include:

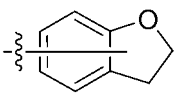, 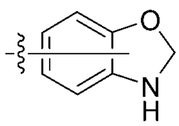, 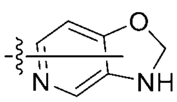, 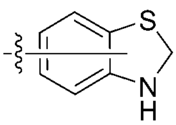,

etc.

The term "alkoxy" refers to -O-(alkyl), wherein the alkyl is as defined above. Non-limiting examples of alkoxy groups include methoxy, ethoxy, propoxy, and butoxy. The term "aryl" refers to a 6- to 14-membered, preferably 6- to 10-membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocycloalkyl, or cycloalkyl ring, wherein the ring attached to the parent structure is the aryl ring; its non-limiting examples include:

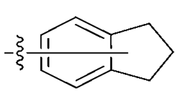, 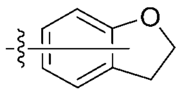, 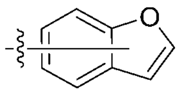, 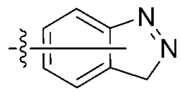, 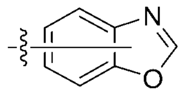,

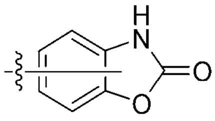, 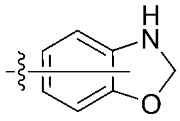, 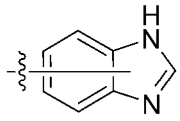, 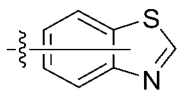, 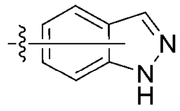,

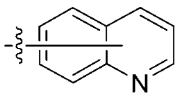, 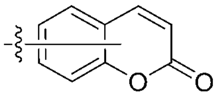, and 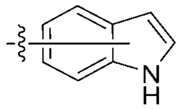.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 6- to 12-membered. More preferably, heteroaryl is 5-membered or 6-membered. For example, its non-limiting examples include imidazolyl, furanyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzimidazolyl,

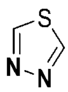, 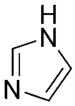, 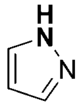,

etc.

The heteroaryl ring may be fused to an aryl, heterocycloalkyl, or cycloalkyl ring, wherein the ring attached to the parent structure is the heteroaryl ring; its non-limiting examples include:

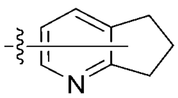, 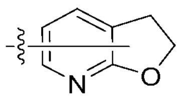, 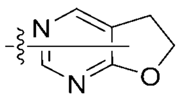, 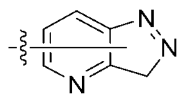, 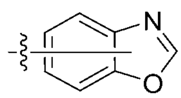,

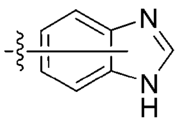, 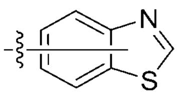, 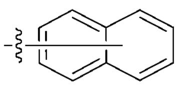, and 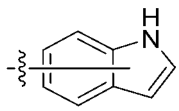.

The term "hydroxy" refers to the -OH group.

The term "halogen" refers to fluorine, chlorine, bromine, or iodine.

The term "cyano" refers to -CN.

The term "amino" refers to $-NH_2$.

The term "nitro" refers to $-NO_2$.

The term "oxo" refers to the =O substituent.

"Substituted" means that one or more, preferably up to 5, and more preferably 1 to 3, hydrogen atoms in the group are independently substituted with a corresponding number of substituents. When the substituent is a ketone or oxo (i.e., =O), two (2) hydrogens on the atom are replaced.

**DETAILED DESCRIPTION**

The preparation of the compounds and pharmaceutically acceptable salts described herein is further described below using examples, but these examples are not intended to limit the scope of the present disclosure.

Experimental methods without specific conditions indicated in the examples of the present disclosure were generally conducted under conventional conditions, or conditions recommended by the manufacturers of the starting materials or commercial products. Reagents without specific sources indicated were commercially available conventional reagents.

The structures of compounds were determined by nuclear magnetic resonance (NMR) spectroscopy and/or mass spectrometry (LCMS). NMR shifts (δ) are given in $10^{-6}$ (ppm). NMR analysis was performed using a Bruker AVANCE-400 nuclear magnetic resonance instrument, with dimethyl sulfoxide-D6 (DMSO-$d_6$), chloroform-D ($CDCl_3$),

and methanol-D4 ($CD_3OD$) as solvents and tetramethylsilane (TMS) as an internal standard. The spatial configurations of the optical isomers (isomers) of compounds can be further confirmed by measuring single-crystal parameters.

HPLC analysis was performed using a Waters ACQUITY ultra high performance LC, Shimadzu LC-20A systems, Shimadzu LC-2010HT series, or Agilent 1200 LC high performance liquid chromatograph (ACQUITY UPLC BEH C18 1.7 μm 2.1 × 50 mm chromatography column, Ultimate XB-C18 3.0 × 150 mm chromatography column, or Xtimate C18 2.1 × 30 mm chromatography column).

MS analysis was performed using a Waters SQD2 mass spectrometer in positive/negative ion scan mode with a mass scan range of 100-1200.

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in thin-layer chromatography separation and purification had a layer thickness of 0.4 mm-0.5 mm.

The flash column purification system used was Combiflash Rf150 (TELEDYNE ISCO) or Isolara one (Biotage).

The normal-phase column chromatography generally used a 100-200 mesh, 200-300 mesh, or 300-400 mesh Yantai Huanghai silica gel as the carrier, or used a Changzhou Santai pre-fill ultrapure normal-phase silica gel column (40-63 μm, 60 Å, 12 g, 25 g, 40 g, 80 g, or other specifications).

The reversed-phase column chromatography generally used a Changzhou Santai pre-fill ultrapure C18 silica gel column (20-45 μm, 100 Å, 40 g, 80 g, 120 g, 220 g, or other specifications).

The high-pressure column purification system used was Waters AutoP equipped with a Waters XBridge BEH C18 OBD Prep Column, 130 Å, 5 μm, 19 mm × 150 mm or Atlantis T3 OBD Prep Column, 100 Å, 5 μm, 19 mm × 150 mm.

The preparative chiral chromatography column used was DAICEL CHIRALPAK IC (250 mm × 30 mm, 10 μm), DAICEL CHIRALCEL OJ (250 mm × 30 mm, 10 μm), or DAICEL CHIRALPAK AD (250 mm × 30 mm, 10 μm).

Known starting materials in the present disclosure may be synthesized using or according to methods known in the art, or may be purchased from Shanghai Titan Scientific, ABCR GmbH & Co. KG, Acros Organics, Aldrich Chemical Company, Accela ChemBio Inc., Darui Chemicals, and other companies.

In the examples, reactions can all be performed in a nitrogen atmosphere unless otherwise specified.

An argon atmosphere or a nitrogen atmosphere means that the reaction flask is connected to a balloon containing about 1 L of argon or nitrogen.

A hydrogen atmosphere means that the reaction flask is connected to a balloon containing about 1 L of hydrogen.

Pressurized hydrogenation reactions were performed using a Parr 3916EKX hydrogenation instrument and a Qinglan QL-500 hydrogen generator, or an HC2-SS hydrogenation instrument.

Hydrogenation reactions generally involved 3 cycles of vacuumization and hydrogen filling.

Microwave reactions were performed using a CEM Discover-S 908860 microwave reactor.

In the examples, solutions were aqueous solutions unless otherwise specified.

In the examples, the temperature of a reaction was room temperature, i.e., 20 °C-30 °C, unless otherwise specified.

The reaction processes in the examples were monitored using thin-layer chromatography (TLC). The developing solvents used for reactions, the eluent systems of column chromatography used for compound purification, and the developing solvent systems of thin-layer chromatography included: A: a dichloromethane/methanol system, B: an n-hexane/ethyl acetate system, C: a petroleum ether/ethyl acetate system, D: a petroleum ether/ethyl acetate/methanol system, and E: a petroleum ether/tetrahydrofuran system. The volume ratio of the solvents was adjusted based on the polarity of the compound, or by adding a small amount of basic or acidic reagents such as triethylamine and acetic acid.

**Example 1**

1-(6-{[(1S,3S)-3-(6,7-Dihydro-5H-cyclopenta[1,2-d]pyrimidin-2-ylamino)cyclopentyl]a mino}pyridin-3-yl)-1,2-dihydropyridin-2-one

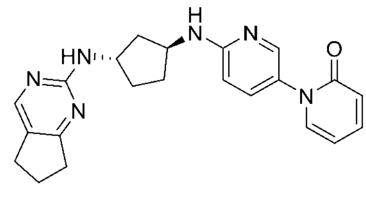

1

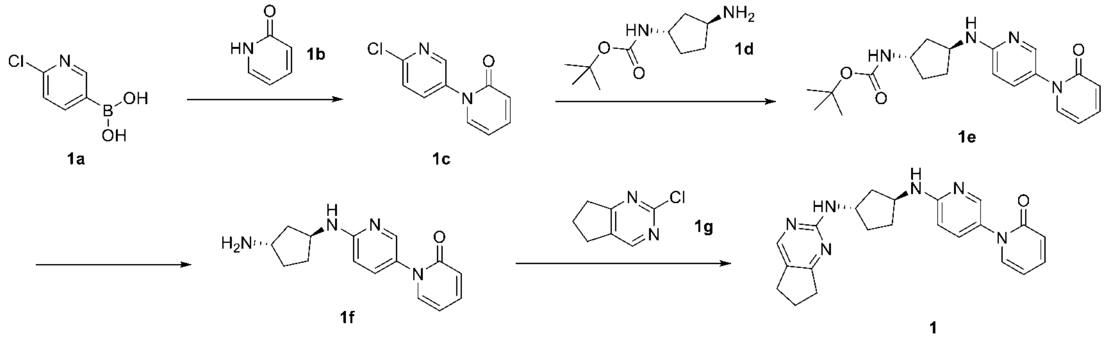


Step 1:

Pyridin-2(1H)-one **1b** (1.51 g, 15.9 mmol), 2-chloro-5-pyridineboronic acid **1a** (5 g, 31.8 mmol), copper acetate (5.77 g, 31.8 mmol), pyridine (2.51 g, 31.8 mmol), and molecular sieves (6 g) were added to a mixed solution (DMF/DCM = 1:6) (210 mL). Air was bubbled through the mixture for one minute, and the mixture was left to react at room temperature for 16 h. The reaction mixture was filtered through diatomaceous earth, and 100 mL of water and 50 mL of ethyl acetate were added to the filtrate. Liquid separation was performed, and the aqueous phase was then extracted with ethyl acetate (50 mL $\times$ 2). The organic phases were combined and concentrated under reduced pressure, and the residue was separated by preparative normal-phase column chromatography to give compound **1c** (1.03 g, yield: 31.4%).

MS m/z (ESI): 207.1 $[M+H]^+$.

Step 2:

Compound **1c** (1.03 g, 4.99 mmol), tert-butyl (1S,3S)-3-aminocyclopentylcarbamate **1d** (1.2 g, 5.98 mmol), cesium carbonate (4.87 g, 14.96 mmol), and methanesulfonato(2-dicyclohexylphosphino-2',6'-diisopropoxy-1,1'-biphenyl)(2-amino-1,1'-biphenyl-2-yl)palladium(II) (0.42 g, 0.5 mmol) were sequentially added to 1,4-dioxane (10 mL), and the mixture was left to react in a microwave reactor at 130 °C for 4 h. The reaction mixture was separated by preparative normal-phase column chromatography to give compound **1e** (440 mg, yield: 23.8%).

MS m/z (ESI): 371.3 $[M+H]^+$.

Step 3:

Compound **1e** (440 mg, 1.2 mmol) was added to dichloromethane (5 mL), and trifluoroacetic acid (2 mL) was then added. The mixture was left to react at room temperature for 1 h and concentrated under reduced pressure to give compound **1f** (800 mg, yield: 135.1%).

MS m/z (ESI): 271.2 $[M+H]^+$.

Step 4:

Compound **1f** (100 mg, 0.2 mmol), 2-chloro-6,7-dihydro-5H-cyclopenta[1,2-d]pyrimidine **1g** (34.1 mg, 0.22 mmol), and potassium carbonate (83.3 mg, 0.6 mmol) were sequentially added to DMSO (1 mL), and the mixture was heated to 110 °C and left to react for 6 h. The reaction mixture was directly separated by preparative reversed-phase column chromatography to give compound 1 (4 mg, yield: 5.1%).

MS m/z (ESI): 389.3 $[M+H]^+$.

$^1H$ NMR (400 MHz, MeOD) δ 8.03 (s, 1H), 7.98 (d, *J* = 2.6 Hz, 1H), 7.67 - 7.51 (m, 3H), 6.73 (d, *J* = 9.1 Hz, 1H), 6.62 (dd, *J* = 9.1, 1.2 Hz, 1H), 6.47 (td, *J* = 6.8, 1.4 Hz, 1H), 4.45 (s, 1H), 4.35 (t, *J* = 6.4 Hz, 1H), 2.88 (dt, *J* = 12.9, 7.3 Hz, 4H), 2.35 - 2.26 (m, 2H), 2.19 - 2.03 (m, 4H), 1.68 (qd, *J* = 8.9, 6.1 Hz, 2H).

**Example 2**

1-(6-{[(1S,3S)-3-[(7,7-Difluoro-6,7-dihydro-5H-cyclopenta[1,2-d]pyrimidin-2-yl)amino ]cyclopentyl]amino}pyridin-3-yl)-1,2-dihydropyridin-2-one

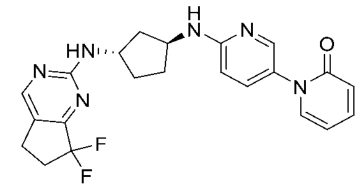

**2**

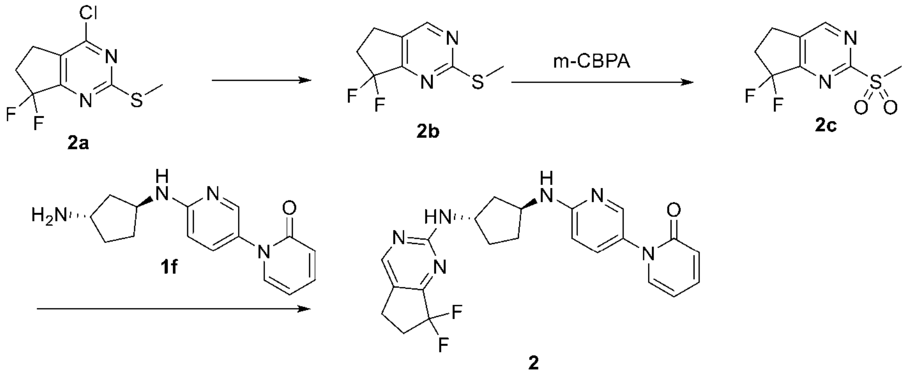


2a 2b 2c 1f 2

Step 1:

In a hydrogen atmosphere, compound **2a** (200 mg, 0.85 mmol, refer to Patent Application No. WO201528848 for the synthesis method) was dissolved in methanol (3 mL), and 10% Pd/C (20 mg) was then added. The mixture was left to react at room temperature for 3 h. The reaction mixture was filtered and concentrated to give a crude product, and the crude product was purified by normal-phase column chromatography to give compound **2b** (66 mg, yield: 38%).

MS m/z (ESI): 203.1 $[M+H]^+$.

Step 2:

Compound **2b** (66 mg, 0.33 mmol) was dissolved in dichloromethane (5 mL), and 3-chloroperoxybenzoic acid (168 mg, 0.98 mmol) was then added. The mixture was left to react at room temperature for 2 h. After the reaction was complete, the reaction mixture was directly concentrated to dryness by rotary evaporation to give a crude product, and the crude product was purified by normal-phase column chromatography to give compound **2c** (45 mg, yield: 59%).

MS m/z (ESI): 235.1 $[M+H]^+$.

Step 3:

Compound **2c** (15 mg, 0.06 mmol) and compound **1f** (34.6 mg, 0.13 mmol) were dissolved in tert-butanol (2 mL), and N,N-diisopropylethylamine (24 μL, 0.12 mmol) was then added. The mixture was left to react in a microwave reactor at 110 °C for 4 h. After the reaction was complete, the reaction mixture was directly concentrated to dryness by rotary evaporation to give a crude product, and the crude product was purified by reversed-phase column chromatography to give compound **2** (1.5 mg, yield: 5%).

MS m/z (ESI): 425.4 [M+H]+.

$^1H$ NMR (400 MHz, MeOD) δ 8.37 (s, 1H), 7.94 (d, J = 2.6 Hz, 1H), 7.66 - 7.55 (m, 2H), 7.44 (dd, J = 9.0, 2.7 Hz, 1H), 6.66 - 6.57 (m, 2H), 6.47 (td, J = 6.8, 1.3 Hz, 1H), 4.53 - 4.44 (m, 1H), 4.40 - 4.31 (m, 1H), 2.91 - 2.82 (m, 2H), 2.61 - 2.46 (m, 2H), 2.36 - 2.20 (m, 2H), 2.10 - 1.95 (m, 2H), 1.70 - 1.54 (m, 2H).

**Example 3**

1-(6-{[(1S,3S)-3-(6,7-Dihydro[1,4]dioxino[3,2-d]pyrimidin-2-ylamino)cyclopentyl]ami no}pyridin-3-yl)-1,2-dihydropyridin-2-one

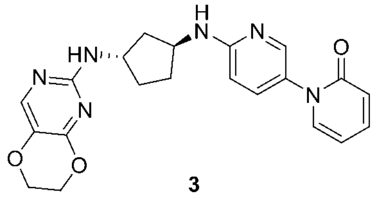

3

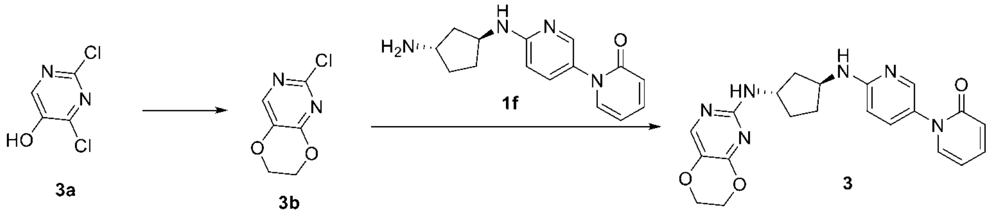

3a 3b 1f 3

Step 1:

2,4-Dichloro-5-hydroxypyrimidine **3a** (520 mg, 3.2 mmol), 2-bromoethanol (1.18 g, 9.5 mmol), and potassium carbonate (2.18 g, 15.8 mmol) were sequentially added to DMF (5 mL). The mixture was left to react in a microwave reactor at 100 °C for 1 h. The reaction mixture was poured into water (15 mL) and extracted with ethyl acetate (20 mL × 2). The organic phases were combined and concentrated under reduced pressure to give a crude product, and the crude product was separated by preparative normal-phase column chromatography to give compound **3b** (120 mg, yield: 22.1%).

MS m/z (ESI): 173.1 $[M+H]^+$.

Step 2:

**3b** (100 mg, 0.37 mmol), compound **1f** (53 mg, 0.24 mmol), cesium carbonate (361.6 mg, 1.11 mmol), and dichloro[1,3-bis(2,6-di-3-pentylphenyl)imidazol-2-ylidene](3-chloropyridyl)palladium(I I) (29.3 mg, 0.04 mmol) were sequentially added to 1,4-dioxane (1 mL). The mixture was left to react in a microwave reactor at 100 °C for 2 h. The reaction mixture was filtered and concentrated to give a crude product, and the crude product was separated by preparative reversed-phase column chromatography to give compound **3** (5 mg, yield: 3.33%).

MS m/z (ESI): 407.3 $[M+H]^+$.

$^1$H NMR (400 MHz, MeOD) δ 7.93 (d, J = 2.7 Hz, 1H), 7.78 (s, 1H), 7.60 (ddt, J = 11.1, 6.7, 2.0 Hz, 2H), 7.44 (dd, J = 9.0, 2.7 Hz, 1H), 6.61 (ddd, J = 9.0, 2.9, 1.7 Hz, 2H), 6.46 (td, J = 6.8, 1.3 Hz, 1H), 4.49 - 4.43 (m, 2H), 4.30 (dp, J = 13.4, 6.5 Hz, 2H), 4.21 - 4.15 (m, 2H), 2.31 - 2.15 (m, 2H), 1.95 (qd, J = 6.5, 2.6 Hz, 2H), 1.63 - 1.51 (m, 2H).

**Example 4**

2-(5-Fluoro-6-{[(1S,3S)-3-(6,7-dihydro[1,4]dioxino[3,2-d]pyrimidin-2-ylamino)cyclope ntyl]amino}pyridin-3-yl)-3H,2H-1,2-pyridazin-3-one

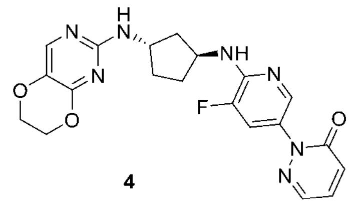

4

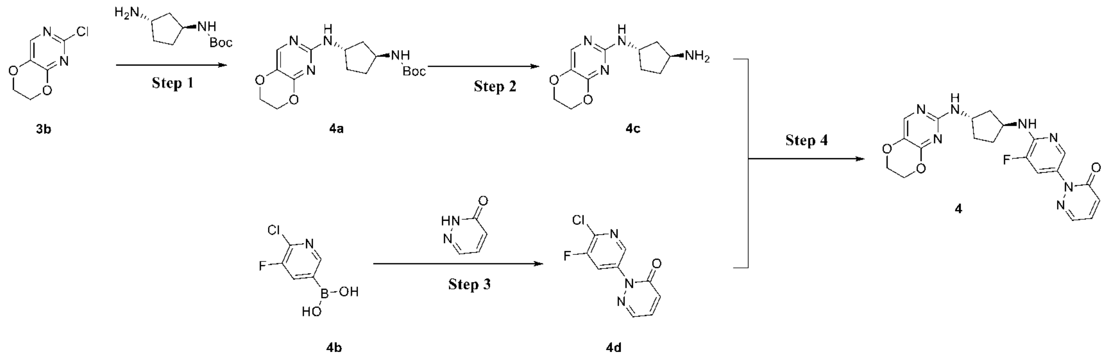


3b 4a 4c 4b 4d 4

Step 1:

Compound **3b** (2 g, 11.6 mmol), tert-butyl (1S,3S)-3-aminocyclopentylcarbamate (2.8 g, 13.92 mmol), cesium carbonate (11.34 g, 34.8 mmol), and dichloro[1,3-bis(2,6-di-3-pentylphenyl)imidazol-2-ylidene](3-chloropyridyl)palladium(I I) (550 mg, 0.70 mmol) were weighed into a reaction flask, and dioxane (20 mL) was added. The mixture was left to react overnight at 110 °C in a nitrogen atmosphere. LCMS analysis showed the formation of the product. The reaction mixture was concentrated and purified by silica gel column chromatography to give compound **4a** (2.5 g, yield: 64.1%).
MS m/z (ESI): 337.2 $[M+H]^+$.

Step 2:

Compound **4a** (2.5 g, 7.4320 mmol) was dissolved in DCM (25 mL), and TFA (25 mL) was added. The mixture was left to react overnight at room temperature, and LCMS analysis showed the formation of the product. The reaction mixture was directly evaporated to dryness to give compound **4c** as a tan oil (5.5 g). The product was directly used in the next step.
MS m/z (ESI): 237.2 $[M+H]^+$.

Step 3:

Pyridazin-3(2H)-one (548 mg, 5.70 mmol) was dissolved in 10 mL of a mixed solution (DMF/DCM = 1:6), and compound **4b** (500 mg, 2.85 mmol), copper acetate (1.0 g, 5.70 mmol), pyridine (0.46 mL, 5.70 mmol), and molecular sieves were then sequentially added. The mixture was stirred at room temperature for 16 h. After the reaction was complete, the reaction mixture was filtered through diatomaceous earth, and 10 mL of water and 30 mL of ethyl acetate were added to the filtrate. The mixture was stirred at room temperature for 15 min and extracted several times with ethyl acetate (20 mL × 3). The organic phases were combined, dried over anhydrous sodium sulfate, and filtered. The filtrate was concentrated under reduced pressure, and the resulting residue was purified by silica gel column chromatography with an eluent system (PE:EA = 5:1 to 1:1) to give compound **4d** (510 mg, yield: 79.3%).
MS m/z (ESI): 226.1 $[M+H]^+$.

Step 4:

Compound **4c** (990 mg, 1.33 mmol), compound **4d** (300 mg, 1.3 mmol), cesium carbonate (6.5 g, 19.9 mmol), and dichloro[1,3-bis(2,6-di-3-pentylphenyl)imidazol-2-ylidene](3-chloropyridyl)palladium(I I) (63.3 mg, 79.78 μmol) were dissolved in dioxane (30 mL). The solution was left to react at 110 °C for 4 h in a nitrogen atmosphere, and LCMS analysis showed the formation of the product. The reaction mixture was cooled to room temperature. Water and ethyl acetate were added, and liquid separation was performed. The aqueous phase was extracted with ethyl acetate, and the organic phases were combined, washed with saturated brine, dried over anhydrous sodium sulfate, and filtered. The filtrate was concentrated and purified by column chromatography to give compound **4** (375 mg, yield: 66.44%).
MS m/z (ESI): 426.2 $[M+H]^+$.
$^1$H NMR(400 MHz, DMSO-$d_6$) δ 8.08-7.99 (m, 2H), 7.83 (s, 1H), 7.62 (m, 1H), 7.48 (m, 1H), 7.05 (m, 1H), 6.91 (m,

1H), 6.76 (m, 1H), 4.49 (m, 1H), 4.43-4.35 (m, 2H), 4.23 (m, 1H), 4.17-4.10 (m, 2H), 2.18-2.01 (m, 2H), 1.96-1.83 (m, 2H), 1.62-1.40 (m, 2H).

**Example 5**

2-(6-{[(1S,3S)-3-(6,7-Dihydro[1,4]dioxino[3,2-d]pyrimidin-2-ylamino)cyclopentyl]ami no}pyridin-3-yl)-3H,2H-1,2-pyridazin-3-one

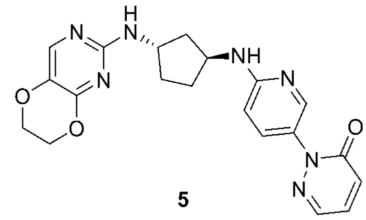

5

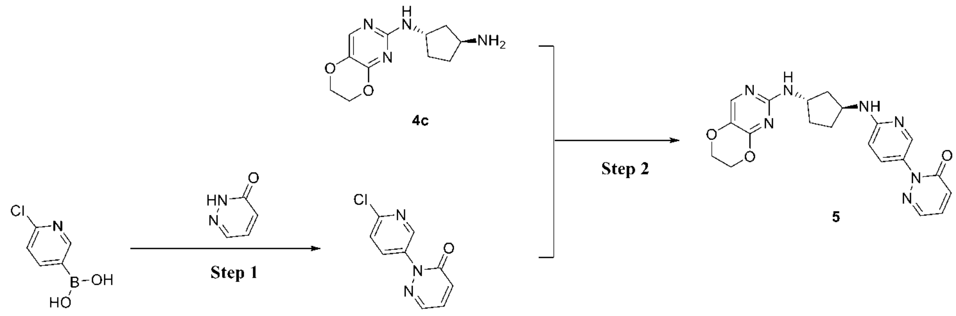


4c

5

5a

5b

Step 1:

Pyridazin-3(2H)-one (1 g, 10.4 mmol) was dissolved in 120 mL of a mixed solution (DMF/DCM = 1:6), and compound **5a** (2.4 g, 15.3 mmol), copper acetate (3.8 g, 20.8 mmol), pyridine (1.6 g, 20.8 mmol), and molecular sieves (4 g) were then sequentially added. The mixture was stirred at room temperature for 16 h. Subsequently, the reaction mixture was filtered through diatomaceous earth, and 10 mL of water and 20 mL of ethyl acetate were added to the filtrate. The mixture was stirred at room temperature for 15 min and extracted several times with ethyl acetate (20 mL × 3). The organic phases were combined, dried over anhydrous sodium sulfate, and filtered. The filtrate was concentrated under reduced pressure, and the resulting residue was purified by silica gel column chromatography with an eluent system (PE:EA = 5:1 to 1:1) to give the title compound **5b** (500 mg, yield: 23.4%).
MS m/z (ESI): 208.3 $[M+H]^+$.

Step 2:

Compound **4c** (1.07 g, 1.45 mmol), compound **5b** (300 mg, 1.45 mmol), cesium carbonate (7.06 g, 21.67 mmol), and dichloro[1,3-bis(2,6-di-3-pentylphenyl)imidazol-2-ylidene](3-chloropyridyl)palladium(I I) (68.8 mg, 86.69 μmol) were dissolved in dioxane (30 mL), and the solution was left to react at 110 °C for 4 h in a nitrogen atmosphere. LCMS analysis showed the formation of the target compound. The reaction mixture was cooled to room temperature and filtered under reduced pressure, and the filtrate was evaporated to dryness. Water and ethyl acetate were added, and liquid separation was performed. The aqueous phase was extracted with ethyl acetate, and the organic phases were combined, washed with saturated brine, dried over anhydrous sodium sulfate, and filtered. The filtrate was concentrated and purified by column chromatography to give compound **5** (300 mg, yield: 48.9%).
MS m/z (ESI): 408.1 $[M+H]^+$.
$^1H$ NMR(400 MHz, DMSO-$d_6$) δ 8.10 (m, 1H), 8.01 (m, 1H), 7.83 (s, 1H), 7.54 - 7.42 (m, 2H), 7.02 (m, 1H), 6.90 (m, 1H), 6.76 (m, 1H), 6.52 (m, 1H), 4.44-4.34 (m, 2H), 4.25 (m, 2H), 4.17-4.11(m, 2H), 2.20-1.99 (m, 2H), 1.91-1.76 (m, 2H), 1.53-1.36 (m, 2H).

**Example 6**

1-(5-Fluoro-6-{[(1S,3S)-3-(6,7-dihydro[1,4]dioxino[3,2-d]pyrimidin-2-ylamino)cyclope ntyl]amino}pyridin-3-yl)-1,2-dihydropyridin-2-one

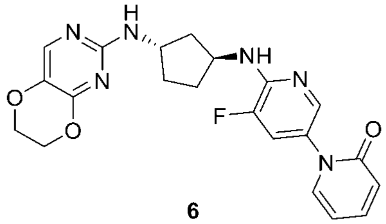

6

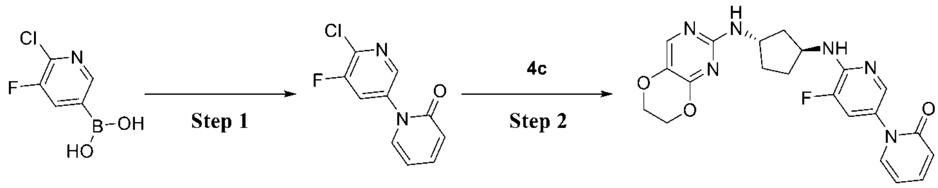


5a 6a 6

Step 1:

Compound **5a** (3 g, 17.11 mmol), pyridin-2(1H)-one (813.49 mg, 8.55 mmol), pyridine (1.35 g, 17.11 mmol), copper acetate (3.1 g, 17.11 mmol), and 4A molecular sieves (3.25 g) were weighed out and dissolved in DCM (60 mL) and DMF (5 mL), and the solution was left to react for 6 h in an oxygen atmosphere. TLC analysis showed that the reaction was complete. The reaction mixture was filtered under reduced pressure, and the filter cake was rinsed with dichloromethane. The organic phases were combined, concentrated, and purified by column chromatography to give compound **6a** (1.8 g, yield: 93.68%).

MS m/z (ESI): 225.1 $[M+H]^+$.

Step 2:

Compound **4c** (2.12 g, 2.85 mmol), compound **6a** (640 mg, 2.85 mmol), cesium carbonate (13.92 g, 42.74 mmol), and dichloro[1,3-bis(2,6-di-3-pentylphenyl)imidazol-2-ylidene](3-chloropyridyl)palladium(I I) (135.68 mg, 170.95 μmol) were weighed into a reaction flask, and dioxane (60 mL) was added. The mixture was left to react at 110 °C for 4 h in a nitrogen atmosphere, and LCMS analysis showed the formation of the target compound. The reaction mixture was cooled to room temperature and filtered under reduced pressure, and the filtrate was evaporated to dryness. Water and ethyl acetate were added, and liquid separation was performed. The aqueous phase was extracted with ethyl acetate, and the organic phases were combined, washed with saturated brine, dried over anhydrous sodium sulfate, and filtered. The filtrate was concentrated and purified by column chromatography to give compound **6** (350 mg, yield: 33.15%).

MS m/z (ESI): 425.1 $[M+H]^+$.

$^1$H NMR(400 MHz, DMSO-$d_6$) δ 7.86 - 7.80 (m, 2H), 7.64 (m, 1H), 7.56-7.43 (m, 2H), 6.88 (m, 1H), 6.75 (m, 1H), 6.46 (m, 1H), 6.28 (m, 1H), 4.47 (m, 1H), 4.42-4.34 (m, 2H), 4.23 (m, 1H), 4.17-4.10 (m, 2H), 2.09 (m, 2H), 1.96-1.81 (m, 2H), 1.65-1.37 (m, 2H).

**Example 7**

1-(5-{[(1S,3S)-3-(6,7-Dihydro[1,4]dioxino[3,2-d]pyrimidin-2-ylamino)cyclopentyl]ami no}pyrazin-2-yl)-1,2-dihydropyridin-2-one

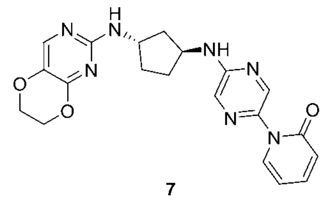

7

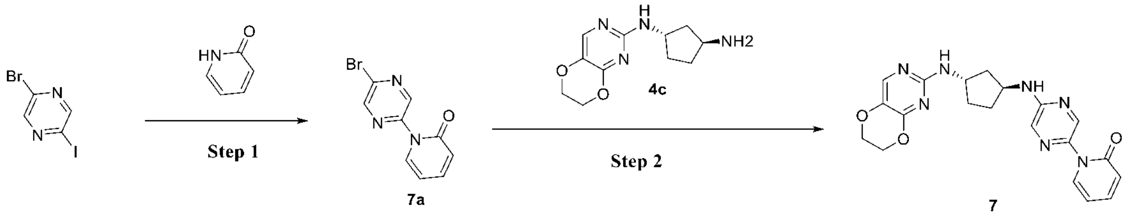


7a 4c 7

Step 1:

2-Bromo-5-chloropyrazine (250 mg, 2.629 mmol), 2-hydroxypyridine (786 mg, 2.760 mmol), (1R,2R)-(-)-N,N'-dimethyl-1,2-cyclohexanediamine (74.48 mg, 0.526 mmol), cuprous iodide (50.07 mg, 0.263 mmol), and potassium carbonate (726.66 mg, 5.258 mmol) were added to a reaction flask, and DMSO (5 mL) was then added. The reaction system was purged three times with nitrogen. The reaction system was left to react in a microwave reactor at 120 °C for two hours. LC-MS analysis showed that the starting materials were completely converted. The reaction mixture was concentrated under reduced pressure and purified by silica gel column chromatography to give compound **7a** (390 mg, yield: 59%).

MS m/z (ESI): 252.1 [M+1].

Step 2:

Compound **4c** (100 mg, 0.191 mmol), compound **7a** (72.17 mg, 0.286 mmol), the Pd-PEPPSI-IPent catalyst (15.1 mg, 0.019 mmol), and cesium carbonate (310.72 mg, 0.954 mmol) were added to a reaction flask, and 1,4-dioxane (1 mL) was then added. The reaction system was purged three times with nitrogen. The reaction system was placed in a 100 °C oil bath and stirred for 5 h. LC-MS analysis showed that the starting materials were completely converted. The reaction mixture was cooled to room temperature, concentrated under reduced pressure, purified by silica gel column chromatography, and purified by reversed-phase high performance liquid chromatography to give the target compound **7** (10 mg).

MS m/z (ESI): 408.5 [M+1].

$^1H$ NMR (400 MHz, DMSO) δ 8.23 (d, *J* = 1.3 Hz, 1H), 7.86 - 7.81 (m, 2H), 7.73 - 7.68 (m, 1H), 7.52 - 7.43 (m, 2H), 6.79 (d, *J* = 7.2 Hz, 1H), 6.46 (dt, *J* = 9.3, 1.1 Hz, 1H), 6.32 (td, *J* = 6.7, 1.4 Hz, 1H), 4.42 - 4.36 (m, 2H), 4.32 - 4.18 (m, 2H), 4.17 - 4.10 (m, 2H), 2.20 - 2.00 (m, 2H), 1.94 - 1.80 (m, 2H), 1.55 - 1.43 (m, 2H).

## Example 8

2-(5-{[(1S,3S)-3-(6,7-Dihydro[1,4]dioxino[3,2-d]pyrimidin-2-ylamino)cyclopentyl]ami no}pyrazin-2-yl)-2,3-dihydro-1H-isoindol-1-one

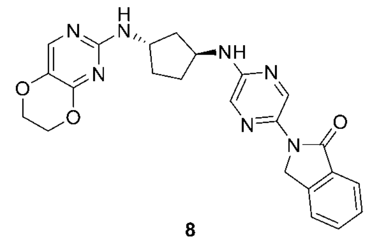

8

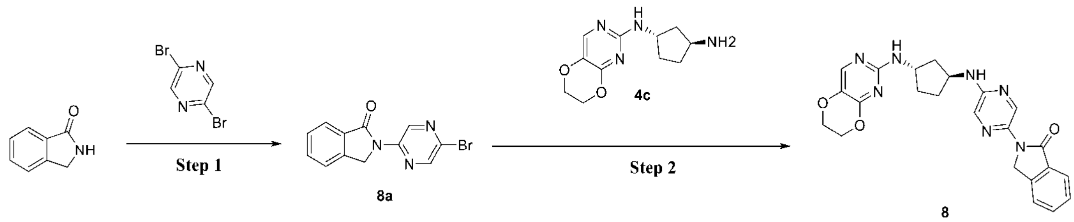


Step 1:

Isoindolin-1-one (300 mg, 2.253 mmol), 2,5-dibromopyrazine (589 mg, 2.478 mmol), (1R,2R)-(-)-N,N'-dimethyl-1,2-cyclohexanediamine (64.10 mg, 0.451 mmol), cuprous iodide (42.91 mg, 0.225 mmol), and potassium phosphate (956.53 mg, 4.506 mmol) were added to a reaction flask, and 1,4-dioxane (6 mL) was added. The reaction system was purged three times with nitrogen. The reaction system was placed in a 100 °C oil bath and stirred overnight. LCMS analysis showed that the starting materials were completely converted. The reaction mixture was cooled to room temperature, concentrated under reduced pressure, and purified by silica gel column chromatography to give compound **8a** (270 mg, 41%).

MS m/z (ESI): 190.0 [M+1].

Step 2:

Compound **8a** (60 mg, 0.210 mmol), compound **4c** (100 mg, 0.191 mmol), the Pd-PEPPSI-IPent catalyst (15.1 mg, 0.019 mmol), and cesium carbonate (310.72 mg, 0.954 mmol) were added to a reaction flask, and 1,4-dioxane (1 mL) was then added. The reaction system was purged three times with nitrogen. The reaction system was placed in a 100 °C oil bath and stirred for 5 h. LCMS analysis showed that the starting materials were completely converted. The reaction mixture was cooled to room temperature, concentrated under reduced pressure, and purified by silica gel column chromatography and prep-HPLC to give compound **8** (23 mg).

MS m/z (ESI): 446.4 [M+1].

1H NMR (400 MHz, DMSO) δ 9.01 (d, *J* = 1.5 Hz, 1H), 7.83 (s, 1H), 7.81 - 7.76 (m, 2H), 7.69 - 7.65 (m, 2H), 7.57 - 7.51 (m, 1H), 7.02 (d, *J* = 6.9 Hz, 1H), 6.78 (d, *J* = 7.2 Hz, 1H), 4.98 (s, 2H), 4.42 - 4.36 (m, 2H), 4.28 - 4.18 (m, 2H), 4.16 - 4.11 (m, 2H), 2.20 - 2.03 (m, 2H), 1.93 - 1.77 (m, 2H), 1.55 - 1.41 (m, 2H).

**Example 9**

3-(6-{[(1S,3S)-3-(6,7-Dihydro[1,4]dioxino[3,2-d]pyrimidin-2-ylamino)cyclopentyl]ami no}pyridin-3-yl)-1-methyl-2-oxotetrahydro-1H-imidazol-4-one

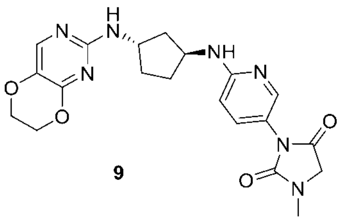

9

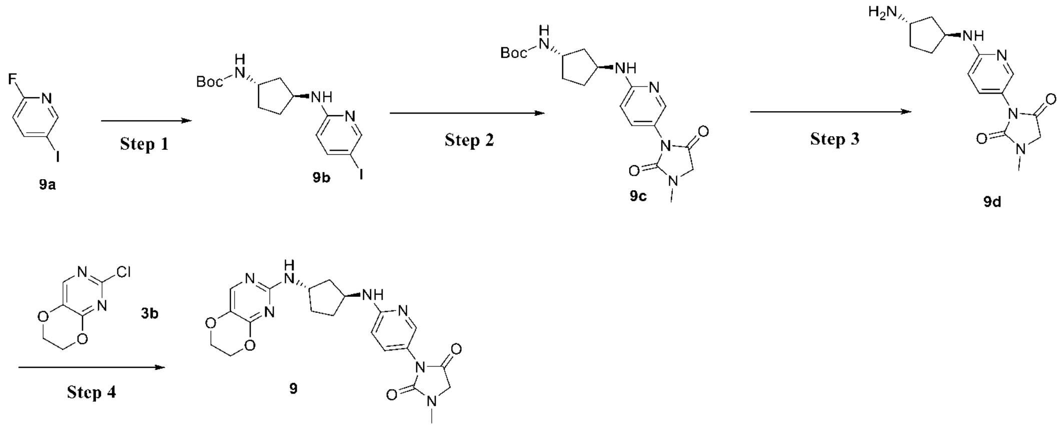


9a 9b 9c 9d 3b 9

Step 1:

2-Fluoro-5-iodopyridine **9a** (5878 mg, 26.4 mmol), tert-butyl ((1S,3S)-3-aminocyclopentyl)carbamate (4400 mg, 21.97 mmol), DIEA (8518 mg, 65.91 mmol), and DMSO (60 mL) were weighed into a reaction flask, and the mixture was left to react at 120 °C for 16 h in a nitrogen atmosphere. LCMS analysis showed that the starting materials were completely consumed. Ethyl acetate (200 mL) was added, and the mixture was washed three times with saturated brine (30 mL) and concentrated under reduced pressure. The residue was purified by column chromatography to give the target compound 9b (5000 mg, yield: 56.4%).
MS m/z (ESI): 404.2 [M+1].

Step 2:

Compound **9b** (500 mg, 1.24 mmol), 1-methylimidazoline-2,4-dione (283 mg, 2.48 mmol), potassium phosphate (790 mg, 3.72 mmol), cuprous iodide (236 mg, 1.24 mmol), trans-N,N'-dimethylcyclohexane-1,2-diamine (212 mg, 1.50 mmol), and isopropanol (10 mL) were weighed into a reaction flask. In a nitrogen atmosphere, the mixture was stirred in a microwave reactor at 110 °C for 5 h. LCMS analysis showed the formation of the product. The reaction mixture was filtered through diatomaceous earth, and the filtrate was concentrated under reduced pressure. Deionized water and dichloromethane were added to the residue, and liquid separation was performed. The organic phase was washed with saturated brine, dried over anhydrous sodium sulfate, and filtered. The filtrate was concentrated under reduced pressure and purified by column chromatography to give compound **9c** (250 mg, yield: 51.8%).
MS m/z (ESI): 390.5 [M+1].

Step 3:

Compound **9c** (259 mg, 0.64 mmol) was dispersed in dichloromethane (5 mL), and 4 N hydrochloric acid in dioxane (5 mL) was added. The mixture was left to react at room temperature for 4 h. LCMS analysis showed that the starting materials were completely consumed. The reaction mixture was concentrated to dryness by rotary evaporation under reduced pressure, and a small amount of methanol was added to dissolve the residue. Ethyl acetate was added, and a large amount of solid precipitated. The mixture was filtered, and the filter cake was washed with ethyl acetate to give a crude product. The crude product was added to DCM (10 mL) in a reaction flask, and 1 mL of triethylamine was added. After 10 min of stirring, the reaction mixture was concentrated to give crude compound **9d** (180 mg).
MS m/z (ESI): 290.5 [M+1].

Step 4:

Compound **9d** (180 mg, 0.62 mmol), compound **3b** (108 mg, 0.62 mmol), cesium carbonate (2026 mg, 6.22

mmol), the Pd-PEPPSI-IPent catalyst (99 mg, 0.12 mmol), and dioxane (10 mL) were weighed out and left to react at 110 °C for 16 h in a nitrogen atmosphere. LCMS analysis showed the formation of the product. The reaction mixture was filtered through diatomaceous earth, and the filtrate was concentrated and purified by pre-HPLC to give compound **9** (12 mg, yield: 4.53%).

MS m/z (ESI): 426.5 [M+1].

$^1$H NMR (400 MHz, DMSO-d6) δ 7.85 - 7.81 (m, 2H), 7.25 (dd, *J* = 8.9, 2.6 Hz, 1H), 6.84 (d, *J* = 6.9 Hz, 1H), 6.75 (d, *J* = 7.2 Hz, 1H), 6.50 (d, *J* = 8.9 Hz, 1H), 4.42 - 4.36 (m, 2H), 4.28 - 4.16 (m, 2H), 4.16 - 4.12 (m, 2H), 4.07 (s, 2H), 2.90 (s, 3H), 2.15 - 1.99 (m, 2H), 1.89 - 1.73 (m, 2H), 1.52 - 1.38 (m, 2H).

## Example 10

2-(6-{[(1S,3S)-3-(6,7-Dihydro[1,4]dioxino[3,2-d]pyrimidin-2-ylamino)cyclopentyl]ami no}pyridin-3-yl)-4-methyl-3H,2H-1,2-pyridazin-3-one

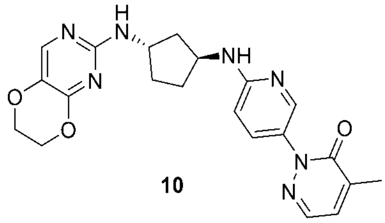

**10**

The compound of Example **10** was obtained by referring to the synthesis scheme of Example **1.**

MS m/z (ESI): 422.5 [M+1].

1H NMR (400 MHz, DMSO-d6) δ 8.08 (d, *J* = 2.4 Hz, 1H), 7.89 (d, *J* = 4.0 Hz, 1H), 7.83 (s, 1H), 7.49 (dd, J= 8.8, 2.8 Hz, 1H), 7.34 (dd,J= 4.0, 1.2 Hz, 1H), 6.87 (d, *J* = 6.8 Hz, 1H), 6.76 (d, *J* = 7.2 Hz, 1H), 6.51 (dd, *J* = 8.8, 0.8 Hz, 1H), 4.41 -4.37 (m, 2H), 4.34 - 4.17 (m, 2H), 4.16 - 4.11 (m, 2H), 2.18 - 2.01 (m, 5H),1.90 -1.76 (m, 2H), 1.53 -1.41 (m, 2H).

## Example 11

1-(6-{[(1S,3S)-3-(Quinazolin-2-ylamino)cyclopentyl]amino}pyridin-3-yl)-1,2-dihydrop yridin-2-one

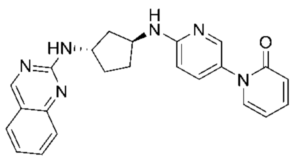

11

The compound of Example **11** was obtained by referring to the synthesis scheme of Example **1.**

MS m/z (ESI): 399.19 [M+1].

$^1$H NMR (400 MHz, MeOD) δ 9.02 (s, 1H), 7.95 (d, *J* = 2.6 Hz, 1H), 7.75 (dd, *J* = 8.1, 1.5 Hz, 1H), 7.70 (ddd, *J* = 8.4, 6.9, 1.5 Hz, 1H), 7.65 - 7.52 (m, 3H), 7.45 (dd, *J* = 9.0, 2.7 Hz, 1H), 7.25 (t, *J* = 7.5 Hz, 1H), 6.63 (t, *J* = 8.9 Hz, 2H), 6.47 (d, *J* = 1.4 Hz, 1H), 4.65 - 4.54 (m, 1H), 4.44 - 4.33 (m, 1H), 2.39 - 2.27 (m, 2H), 2.14 - 2.03 (m, 2H), 1.75 - 1.59 (m, 2H).

## Bioactivity Assays

The present disclosure is further described and explained below with reference to test examples. However, these examples are not intended to limit the scope of the present disclosure.

## Test Example 1. Assessment of Binding of Compounds of Present Disclosure to PCSK9

Table 1. Main experimental materials and instruments

| Name | Supplier |
|---|---|
| PCSK9 (biotinylated) | Arco |
| SA chip | Cytiva |
| U96 microplate | Greiner |
| SPR device | BIAcore S200 |
| Centrifuge | Eppendorf |

**1. Experimental steps**

**1.1. Reagent preparation**

1) Protein: PCSK9 (biotinylated) (Acro).
The protein was dissolved in water to 200 μg/mL, and the solution was stored at -80 °C.
2) Chip: SA chip.
3) Running buffer: 1 × HBSP + 4% DMSO + 0.1 mM CaCl2.

**1.2. Protein immobilization**

1) The chip was pre-warmed to room temperature.
2) The required buffer was prepared and filtered through a 0.22 μm filter membrane.
3) The chip was loaded, and parameters were written to the chip.
4) The PCSK9 protein was diluted to 50 μg/mL in PBS.
5) An immobilization program was set on the BIAcore S200 instrument.
6) The immobilization program was run.
7) The final immobilization response value was 8000 RU, and the maximum response value was 49.4 RU.

**1.3. Compound testing**

1) The compounds were serially diluted 3-fold in the running buffer. The reference compound was diluted 3-fold from 0.4 μM. The test compounds were diluted 3-fold from 10 μM.
2) A dissolution correction buffer was prepared according to the table below:

Table 2. Dissolution correction buffer

| Buffer/volume | 1 | 2 | 3 | 4 | 5 | 6 | 7 | 8 |
|---|---|---|---|---|---|---|---|---|
| 3.5% DMSO | 0 | 200 | 400 | 600 | 800 | 1000 | 1200 | 1400 |
| 4.8% DMSO | 1400 | 1200 | 1000 | 800 | 600 | 400 | 200 | 0 |

3) The "LMW multi kinetics" program of the instrument was set as a running program for compound testing.

a) The association time was set at 100 s, and the dissociation time was set at 300 s.

4) The compound testing program was run.

**1.4. Data analysis**

1)

$$Rmax = (MW\ analyte / MW\ ligand) \times RL \times Sm$$

When affinity fitting was used to fit a curve, the response value of the highest concentration should be close to the fitted Rmax value for an optimal result.
2) Solvent correction and compound concentration gradients were checked.
3) The baseline, binding, and positive compound results in the test report were checked.
4) A suitable fitting method was selected according to the actual association to fit a curve.
5) Whether the fitting result met the data quality assessment criteria of the instrument was checked.

Table 3. The binding of compounds of the present disclosure to PCSK9 ($K_D$)

| Example | $K_D$ (nM) |
|---|---|
| 1 | 26.3 |
| 2 | 68.7 |
| 3 | 75.8 |
| 11 | 180.0 |

According to the results, the binding of Examples **1-3** was stronger than that of Example **11,** the binding of Example **3** was more than 10 times stronger than that of the reference compound **2,** and the binding of Example **3** was comparable to that of Examples **7-8.**

**Reference compound 2**

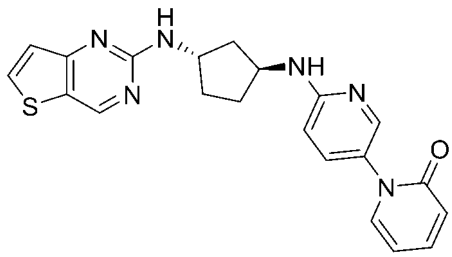

was Example **487** in Patent Application No. WO2020150473 and Patent Application No. WO2020150474.

**Test Example 2. *In Vivo* Pharmacokinetic Experiment in Mice**

C57BL6J mice were used as test animals. After the mice were intragastrically given reference compound **1** (Example 458B in WO2020150473 and WO2020150474) and an example compound, the plasma concentrations of the compounds at different time points were determined by LC/MS/MS. The pharmacokinetic behavior of reference compound **1** and the example compound in mice was studied, and their pharmacokinetic profiles were evaluated.

Test animals: two healthy male mice aged 6-8 weeks (20-30 g) per group
Compound solution preparation: A certain amount of the compound was weighed out and dissolved in a vehicle (5% DMSO/40% PEG400/55% normal saline) to form a 1 mg/mL colorless, clear solution.
Dosing: Mice were fasted overnight and then intragastrically dosed. Reference compound **1** and the example compound were both administered at a dose of 10 mg/kg. Procedure: Mice were intragastrically dosed with reference compound **1** and the example compound. At 0.25 h, 0.5 h, 1 h, 2 h, 4 h, 6 h, 8 h, and 24 h post-dose, blood samples (about 0.03 mL) were collected via peripheral venipuncture, placed into test tubes containing EDTA-K2, and centrifuged at around 4 °C at 4000 g per minute for 5 min to separate plasma, and the plasma was stored at -75 °C.

Determination of the content of the test compound in mouse plasma after the compound (at different concentrations) was orally administered: 10 μL of the mouse plasma collected at each time point post-dose was vortexed with 5 μL of a blank solution and 200 μL of a solution of dexamethasone (internal standard) in acetonitrile for 30 s. The mixture was centrifuged for 15 min (3900 rpm), and 12 μL of the supernatant was taken for LC/MS/MS analysis.

Table 4. Pharmacokinetic parameters of reference compound **1** and Example **3**

| Compound | Reference compound **1** | Example **3** |
|---|---|---|
| $T_{1/2}$ (h) | 1.84 | 2.75 |
| $C_{max}$ (ng/mL) | 11200 | 11450 |
| $AUC_{last}$ (h*ng/mL) | 16475 | 47967 |
| $AUC_{last}$/D (h*mg/mL) | 1647 | 4797 |

Note: All the shown values were means of two animals. Reference compound 1 was Example 458B in Patent Application No. WO2020150473 and Patent Application No. WO2020150474.

According to the results, the oral PK profile of Example **3** in mice was superior-its plasma exposure level was nearly 3 times that of reference compound **1,** and its half-life was longer, indicating that the pharmacokinetic properties of the compound of the present disclosure were superior to those of reference compound **1.**

**Test Example 3. Effects of Reference Compound and Example Compounds on LDLR Level of HepG2 Cells**

Table 5. Main experimental materials and instruments

| Name of reagent | Manufacturer | Cat. No. |
|---|---|---|
| DMEM (4.5 g/L) complete culture medium | Gibco | 11995040 |
| Lipid-depleted fetal bovine serum | VivaCell | C3840-0100 |
| PCSK9 D374Y | Sino biological | 29698-H08H1 |
| Human LDLR ELISA kit | R&D | P373038 |
| HepG2 cells | ATCC | HB-8065 |
| 96-well assay plate | Corning | 3610 |

**Experimental steps**

HepG2 cells were seeded in a 96-well plate at 30,000 cells/well. The next day, 4 nM PCSK9 D374Y was added to each well to stimulate cells, and the cells were treated with a test compound at the same concentration. Three duplicate wells were set for each sample. The cells were cultured at 37 °C with 5% carbon dioxide for 48 h. The cells were washed twice with PBS, and the supernatant was discarded. The cells were then placed on ice and treated with an RIPA lysis buffer containing protease and phosphatase inhibitors at 50 μL/well. The cell culture plate was frozen overnight in a -80 °C freezer, thawed, then lysed on ice for 30 min, and then centrifuged at a low temperature for 15 min. The cell lysate was diluted 20-fold, and an ELISA was performed. A sample or a reference standard was added to a coated well plate at 50 μL/well, and the plate was incubated at room temperature for 2 h. The plate was washed 4 times with a washing buffer by aspiration, and a human LDLR conjugated antibody was added at 200 μL/well. The plate was incubated at room temperature for 2 h. The washing step was repeated, and a substrate solution was added at 200 μL/well. After 30 min, a stop solution was added at 50 μL/well, and the OD was measured at 450 nm. After the LDLR concentration in the sample wells was calculated according to a standard curve, the LDLR increase rate was calculated using the formula (sample value - mean of control wells) / mean of control wells. Data were processed using GraphPad Prism 8.

Table 6. The effects of reference compound **1** and example compounds at a concentration of 75 μM on the LDLR protein level of HepG2 cells

| Compound | LDLR up-regulation level" (% control) |
|---|---|
| Reference compound **1** | 127 |
| Example **3** | 160 |
| Example **5** | 188 |
| Example **6** | 155 |

Note: [a] represents a mean of three duplicate wells. Reference compound **1** was Example **458B** in Patent Application No. WO2020150473 and Patent Application No. WO2020150474.

The effects of the compounds of Examples 7 and 8 at a concentration of 75 μM on the LDLR protein level of HepG2 cells were determined using the same method. The result of the compound of Example 7 was 1.16 times that of reference compound **1,** and the result of the compound of Example 8 was 1.04 times that of reference compound **1.**

According to the results, reference compound **1** and the compounds of Examples **3, 5, 6, 7,** and **8** were all able to up-regulate the LDLR level of HepG2 cells, with the compounds of Examples **3** and **5** showing greater LDLR up-regulation.

**Test Example 4. Metabolite Testing of Reference Compound 1 and Compound of Example 3 in Mouse Hepatocytes**

**1. Objective**

The objective of this experiment was to identify possible metabolites of a test compound in hepatocytes of different species and speculate about its possible metabolic pathway. The test concentration of the test compound was 10 μM.

**2. Materials and reagents**

Mouse hepatocytes were preserved in liquid nitrogen. The specific information is shown in the table below.

**3. Experimental design**

| Species | Strain | Sex | Supplier |
|---|---|---|---|
| Mice | ICR/CD-1 | Male | BioIVT |

**3.1.** Preparation of working solutions of compounds

The test compound and the control drug verapamil powder were dissolved in DMSO to prepare high-concentration stock solutions. Before use, the stock solutions were diluted with DMSO to prepare 2 mM working solutions. The final concentrations of the test compound and verapamil were 10 μM.

**3.2.** Preparation of hepatocyte suspension

1) Before use, the hepatocyte thawing solution and the incubation solution were placed in a 37 °C water bath and preheated for at least 15 min.
2) A tube of cryopreserved hepatocytes was taken. It was ensured that the hepatocytes were still in a low-temperature frozen state before thawing. The hepatocytes were quickly placed in a 37 °C water bath, gently shaken until all ice crystals were dispersed, sprayed with 70% ethanol, and then transferred to a biosafety cabinet.
3) The contents of the small tube of hepatocytes were poured into a centrifuge tube containing 50 mL of thawing culture medium and centrifuged at 100 g for 10 min. After centrifugation, the thawing culture medium was pipetted off, and a sufficient amount of incubation culture medium was added to obtain a cell suspension with a cell density of about $1.5 \times 10^6$ cells/mL.
4) Hepatocytes were counted using Cellometer Vision, and the viable cell density was determined. The hepatocyte suspension was diluted with the incubation culture medium to a viable cell density of $1 \times 10^6$ cells/mL.

**3.3.** Test method

1) 1 μL of the 2 mM test compound solution was pipetted into a 24-well incubation plate, and 199 μL of the hepatocyte suspension was then added to start a reaction. Time points of 0 h, 2 h, and 4 h were set. Different time points corresponded to different wells. The incubation plate was placed back on the vortexer in the incubator (37 °C, 5% $CO_2$, relative humidity 90-95%) and incubated at 500 rpm.

2) At the corresponding time points of incubation, 400 μL of cold acetonitrile containing 0.1% formic acid was immediately added to stop the reaction. After thorough mixing, the mixture was transferred to a corresponding EP tube.
3) The control drug verapamil was incubated and treated according to the method described above, and only 0-hour and 4-hour samples were prepared. The remaining percentage of verapamil at 4 h would be used for determining enzyme activity.
4) All samples were vortexed for 100 s and then centrifuged at 16,000 g for 15 min for protein precipitation. 60 μL of the supernatant was well mixed with 60 μL of purified water, and the mixture was then analyzed by UHPLC-MS/MS.

**4.** Instrument configuration

A Vanquish ultra-high performance liquid chromatograph (Thermo Fisher Scientific, USA), combined with a Thermo Scientific Q Exactive mass spectrometer (Thermo Fisher Scientific, USA) and equipped with an HESI ion source.

**5.** Mass spectrometry analysis

Both UHPLC-MS/MS data acquisition and processing used a Q Exactive high resolution mass spectrometer. An MS full scan was used to trigger MS/MS product ion scans of data-dependent acquisition (DDA).
The related mass spectrometry parameters for the test compound were optimized according to its own nature.

**6.** Data analysis

Both data processing and analysis were performed on Xcalibur (v3.0/4.1, Thermo Fisher Scientific) software, Compound Discoverer 3.0 (Thermo Fisher Scientific) software, and Microsoft Excel 2016.

Table 7. The parent drug remaining proportions (%) of reference compound **1** and Example **3**

| Compound | Retention time (min) | Normalized parent drug remaining proportion (%) after 240 min of incubation |
|---|---|---|
| Reference compound **1** | 8.57 | 42.5% |
| Example **3** | 9.29 | 98.3% |
| Note: Reference compound 1 was Example 458B in Patent Application No. WO2020150473 and Patent Application No. WO2020150474. | | |

According to the experimental results, the stability of the compound of Example 3 in mouse hepatocytes was superior to that of reference compound 1.

**Test Example 5. Stability Test of Test Compounds in Hepatocytes**

**1. Experimental steps**

1) The test compounds and the control drug verapamil powder were dissolved in DMSO to prepare high-concentration stock solutions. Before use, the stock solutions were diluted with DMSO to prepare 100 μM working solutions. The final concentrations of the test compounds and verapamil were 1 μM.
2) A tube of cryopreserved hepatocytes was taken. It was ensured that the hepatocytes were still in a low-temperature frozen state before thawing. The hepatocytes were quickly placed in a 37 °C water bath, gently shaken until all ice crystals were dispersed, sprayed with 70% ethanol, and then transferred to a biosafety cabinet.
3) The contents of small tubes of hepatocytes from different species were poured into a centrifuge tube containing 50 mL of thawing culture medium and centrifuged at 100 g for 10 min. After centrifugation, the thawing culture medium was pipetted off, and a sufficient amount of incubation culture medium was added to obtain a cell suspension with a cell density of about $1.0 \times 10^6$ cells/mL.
4) Hepatocytes were counted using Cellometer Vision, and the viable cell density was determined. The viability of hepatocytes must be greater than 75%. The hepatocyte suspension was diluted with the incubation culture medium to a viable cell density of $0.5 \times 10^6$ cells/mL.
5) 198 μL of the viable cell suspension was transferred to a 96-well deep-well plate, and the deep-well plate was

placed on a vortexer and pre-heated in an incubator for 10 min. In the experiment, the incubation was performed in duplicate.

6) 2 μL of the 100 μM test compound or verapamil solution was added to each well to start a reaction, and the deep-well plate was placed back on the vortexer in the incubator.

7) The samples were incubated. At 0 min, 15 min, 30 min, 60 min, 90 min, and 120 min, 25 μL of the suspension was taken, and 150 μL of acetonitrile containing an internal standard was added to stop the reaction. After 10 min of vortexing, the mixture was centrifuged at 3220 g at 4 °C for 45 min. 100 μL of the supernatant was transferred to a sample injection plate, and 100 μL of purified water was added. After thorough mixing, the mixture was analyzed by UPLC-MS/MS.

**2. Data analysis**

All data calculations were performed through Microsoft Excel software. The peak areas were determined through an extracted ion chromatogram. Through linear fitting of the natural logarithm of the parent drug elimination percentage versus time, the *in vitro* half-life ($T_{1/2}$) of the parent drug was determined.

The *in vitro* half-life ($T_{1/2}$) was calculated through the slope:

$$\textit{In vitro}\ T_{1/2} = 0.693 / k$$

The *in vitro* clearance rate (in μL/min/$10^6$ cells) was calculated using the following formula:

$$\textit{In vitro}\ CL_{int} = kV / N,$$

where V = the incubation volume per well (0.2 mL);
N = the number of cells per well ($0.1 \times 10^6$ cells).

Table 8. The metabolic clearance rates of test compounds in mouse hepatocytes

| Compound | $CL_{int}$ (μL/min/$10^6$ cells) |
|---|---|
| **1** | 13.25 |
| **2** | 20.97 |
| **3** | <0.1 |
| **Reference compound 1** | 6.64 |
| **Reference compound 2** | 15.7 |

According to the results, the stability of Example **3** in mouse hepatocytes was superior to that of the compounds of Examples **1-2, reference compound 1,** and **reference compound 2.**

**Claims**

**1.** A compound represented by formula (I) or a pharmaceutically acceptable salt thereof,

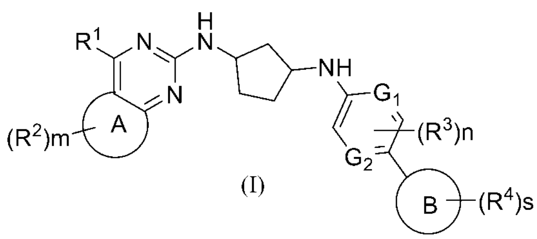

(I)

wherein:

$R^1$ is selected from the group consisting of hydrogen and halogen;

ring A is selected from the group consisting of 3- to 7-membered cycloalkyl and 3- to 7-membered heterocycloalkyl, and the heterocycloalkyl comprises at least one heteroatom selected from the group consisting of O, N, and S;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, and the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$;
or two $R^2$ attached to the same atom, together with the atom to which they are attached, form 3- to 6-membered cycloalkyl or 3- to 6-membered heterocycloalkyl, and the 3- to 6-membered cycloalkyl or 3- to 6-membered heterocycloalkyl is optionally substituted with one or more $R^A$;
$R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
$G_1$ is selected from the group consisting of -N- and $-CR^{3A}-$;
$G_2$ is selected from the group consisting of -N- and $-CR^{3B}-$;
$R^{3A}$, $R^{3B}$, and $R^3$ are each independently selected from the group consisting of hydrogen, halogen, hydroxy, cyano, $C_{1-6}$ alkyl, $C_{1-6}$ alkoxy, $C_{1-6}$ haloalkyl, $C_{1-6}$ haloalkoxy, 3- to 6-membered cycloalkyl, and 4- to 10-membered heterocycloalkyl, and the heterocycloalkyl comprises 1, 2, or 3 heteroatoms selected from the group consisting of O and N, -CONR'R", or -NH-C(O)-OR'; the $C_{1-6}$ alkyl, $C_{1-6}$ alkoxy, $C_{1-6}$ haloalkyl, $C_{1-6}$ haloalkoxy, 3- to 6-membered cycloalkyl, or 4- to 10-membered heterocycloalkyl is optionally substituted with one or more $R^B$, and $R^B$ is selected from the group consisting of halogen, hydroxy, cyano, $C_{1-6}$ alkyl, $C_{1-6}$ haloalkyl, $C_{1-6}$ alkoxy, and 3- to 6-membered cycloalkyl;
R' and R" are each independently selected from the group consisting of hydrogen, $C_{1-6}$ alkyl, and $C_{1-6}$ haloalkyl;
ring B is selected from the group consisting of 5- to 6-membered heterocycloalkyl and 5- to 6-membered heteroaryl, and the 5- to 6-membered heterocycloalkyl or 5- to 6-membered heteroaryl optionally comprises at least one nitrogen atom and/or at least one oxo group;
each $R^4$ is independently selected from the group consisting of halogen, hydroxy, cyano, $C_{1-6}$ alkyl, $C_{1-6}$ alkoxy, $C_{1-6}$ haloalkyl, $C_{1-6}$ haloalkoxy, 3- to 6-membered cycloalkyl, and 4- to 10-membered heterocycloalkyl, and the heterocycloalkyl comprises 1, 2, or 3 heteroatoms selected from the group consisting of O and N; the $C_{1-6}$ alkyl, $C_{1-6}$ alkoxy, $C_{1-6}$ haloalkyl, $C_{1-6}$ haloalkoxy, 3- to 6-membered cycloalkyl, or 4- to 10-membered heterocycloalkyl is optionally substituted with one or more $R^C$, and $R^C$ is selected from the group consisting of halogen, hydroxy, cyano, $C_{1-6}$ alkyl, $C_{1-6}$ haloalkyl, $C_{1-6}$ alkoxy, and 3- to 6-membered cycloalkyl;
m is selected from the group consisting of 0, 1, 2, and 3;
n is selected from the group consisting of 0, 1, 2, and 3; and
s is selected from the group consisting of 0, 1, 2, and 3.

**2.** The compound or the pharmaceutically acceptable salt thereof according to claim 1, wherein ring A is 3- to 7-membered cycloalkyl (e.g., cyclopropyl, cyclobutyl, cyclopentyl, cyclohexyl, or cycloheptyl), preferably cyclopentyl.

**3.** The compound or the pharmaceutically acceptable salt thereof according to claim 1, wherein ring A is 3- to 7-membered heterocycloalkyl (e.g., 3-membered, 4-membered, 5-membered, 6-membered, or 7-membered), and the heterocycloalkyl comprises at least one heteroatom selected from the group consisting of O, N, and S; preferably, ring A is 4- to 6-membered heterocycloalkyl, and the heterocycloalkyl comprises one or two heteroatoms selected from the group consisting of O, N, and S; most preferably, ring A is selected from the group consisting of 5- to 6-membered heterocycloalkyl, and the heterocycloalkyl comprises one or two O atoms.

**4.** The compound or the pharmaceutically acceptable salt thereof according to any one of claims 1 to 3, being a compound represented by formula (I-1) or formula (I-2) or a pharmaceutically acceptable salt thereof:

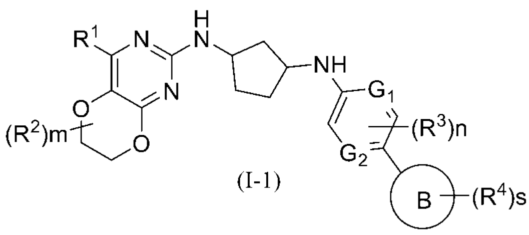

(I-1)

,

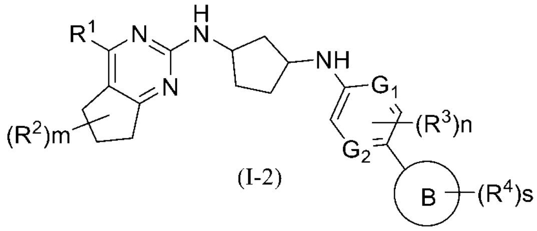

(I-2)

wherein ring B, $R^1$, $R^2$, $G_1$, $G_2$, $R^3$, $R^4$, m, n, and s are as defined in claim 1.

**5.** The compound or the pharmaceutically acceptable salt thereof according to any one of claims 1 to 4, wherein $G_1$ is -N-, and $G_2$ is -$CR^{3B}$-.

**6.** The compound or the pharmaceutically acceptable salt thereof according to any one of claims 1 to 4, wherein $G_1$ is -N-, and $G_2$ is -N-.

**7.** The compound or the pharmaceutically acceptable salt thereof according to any one of claims 1 to 4, wherein $G_1$ is -$CR^{3A}$-, and $G_2$ is -$CR^{3B}$-.

**8.** The compound or the pharmaceutically acceptable salt thereof according to any one of claims 1 to 7, wherein ring B is selected from the group consisting of:

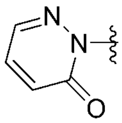 , 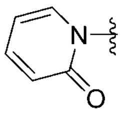 , 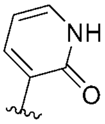 , 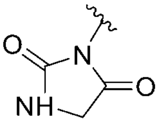 , 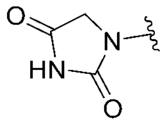 , 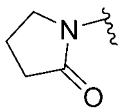 , 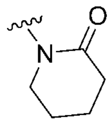 ,

and

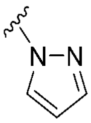 ,

preferably

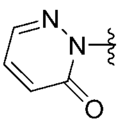 , 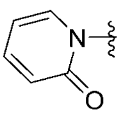 , 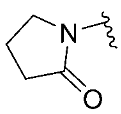 , 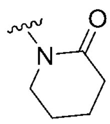 , and 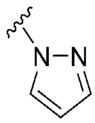 .

**9.** The compound or the pharmaceutically acceptable salt thereof according to any one of claims 1 to 8, wherein each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano; preferably, each $R^4$ is independently selected from the group consisting of fluorine, chlorine, cyano, methyl, trifluoromethyl, trifluoromethoxy, cyclopropyl, and cyanomethyl.

**10.** The compound or the pharmaceutically acceptable salt thereof according to any one of claims 1 to 9, wherein $R^{3A}$, $R^{3B}$, and $R^3$ are each independently selected from the group consisting of hydrogen, halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano; preferably, $R^{3A}$, $R^{3B}$, and $R^3$ are each independently selected from the group consisting of hydrogen, fluorine, chlorine, cyano, methyl, trifluoromethyl, trifluoromethoxy, cyclopropyl, and cyanomethyl.

**11.** The compound or the pharmaceutically acceptable salt thereof according to any one of claims 1 to 5 and 8 to 10, being a compound represented by formula (II-A-1), (II-A-2), (II-A-3), (II-A-4), or (II-A-5) or a pharmaceutically acceptable salt thereof:

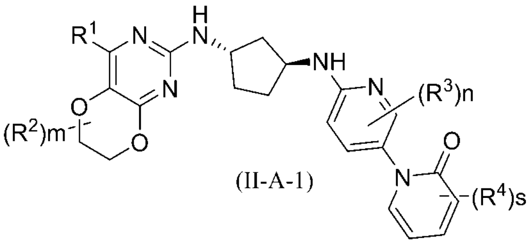
(II-A-1)
,
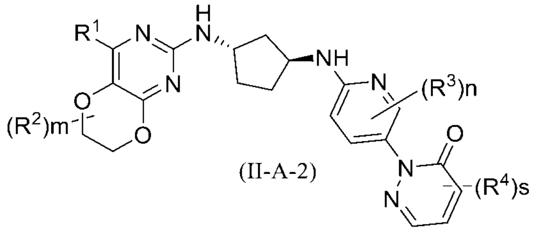
(II-A-2)
,
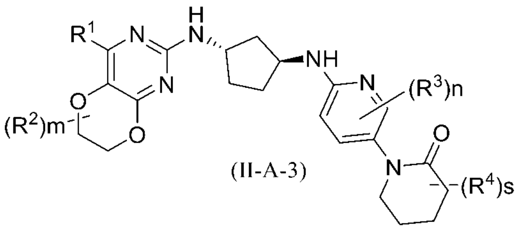
(II-A-3)
,
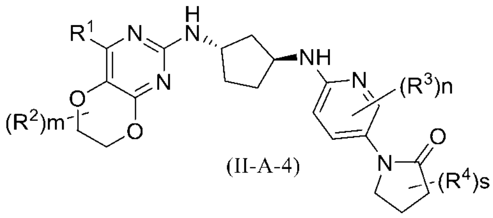
(II-A-4)
, or
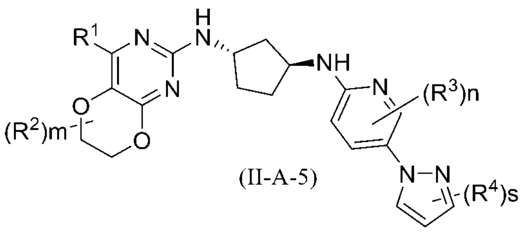
(II-A-5)
,

wherein $R^1$, $R^2$, $R^3$, $R^4$, m, n, and s are as defined in claim 1.

**12.** The compound or the pharmaceutically acceptable salt thereof according to claim 11, wherein:

$R^1$ is hydrogen;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$, and $R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3; preferably,
each $R^3$ is independently selected from the group consisting of fluorine, chlorine, cyano, methyl, trifluoromethyl, and trifluoromethoxy;
each $R^4$ is independently selected from the group consisting of fluorine, chlorine, cyano, methyl, trifluoromethyl, trifluoromethoxy, cyclopropyl, and cyanomethyl;
m is 0;
n and s are each independently selected from the group consisting of 0, 1, and 2.

**13.** The compound or the pharmaceutically acceptable salt thereof according to any one of claims 1 to 5 and 8 to 10, being a compound represented by formula (II-B-1), (II-B-2), (II-B-3), (II-B-4), or (II-B-5) or a pharmaceutically acceptable salt thereof:

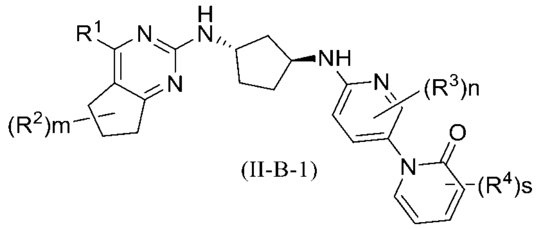
(II-B-1)
,

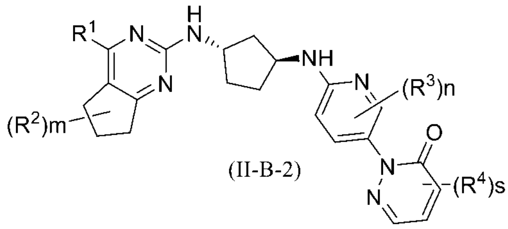
(II-B-2)
,

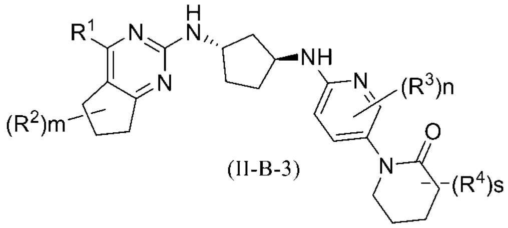
(II-B-3)
,

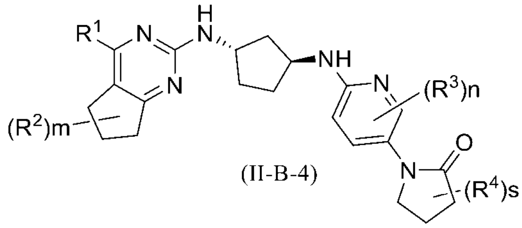
(II-B-4)
, or

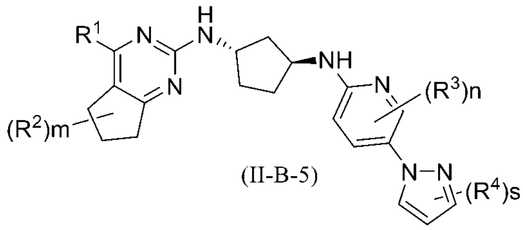
(II-B-5)
,

wherein $R^1$, $R^2$, $R^3$, $R^4$, m, n, and s are as defined in claim 1.

**14.** The compound or the pharmaceutically acceptable salt thereof according to claim 13, wherein:

$R^1$ is hydrogen;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, and the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$; or two $R^2$ attached to the same carbon atom, together with the carbon atom to which they are attached, form 3- to 6-membered cycloalkyl, and the 3-to 6-membered cycloalkyl is optionally substituted with one or more $R^A$;
$R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3; or
preferably,
each $R^2$ is independently selected from the group consisting of fluorine and methyl; or
two $R^2$ attached to the same carbon atom, together with the carbon atom to which they are attached, form cyclopropyl, wherein the cyclopropyl is optionally substituted with one or more $R^A$, and $R^A$ is a halogen;
each $R^3$ is independently selected from the group consisting of fluorine, chlorine, cyano, methyl, trifluoromethyl, and trifluoromethoxy;

each $R^4$ is independently selected from the group consisting of fluorine, chlorine, cyano, methyl, trifluoromethyl, trifluoromethoxy, cyclopropyl, and cyanomethyl;
m, n, and s are each independently selected from the group consisting of 0, 1, 2, and 3.

**15.** The compound or the pharmaceutically acceptable salt thereof according to any one of claims 1 to 4, 6, and 8 to 10, being a compound represented by formula (II-C-1), (II-C-2), (II-C-3), or (II-C-4) or a pharmaceutically acceptable salt thereof:

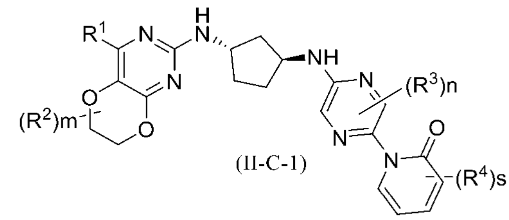
(II-C-1)
,
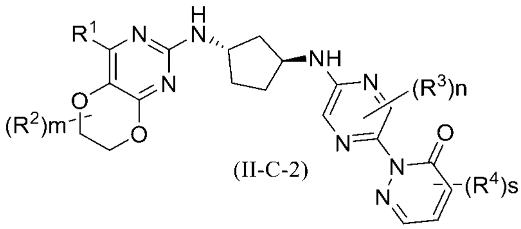
(II-C-2)
,
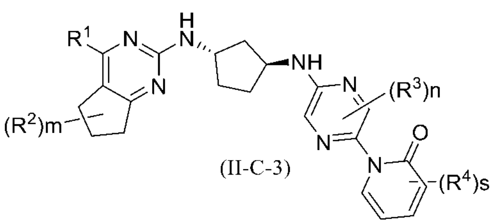
(II-C-3)
, or
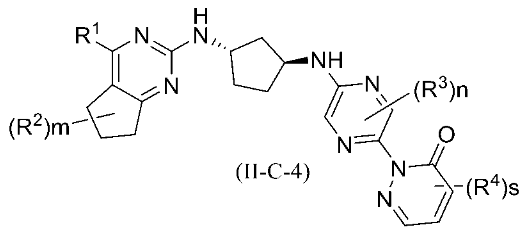
(II-C-4)
,

wherein $R^1$, $R^2$, $R^3$, $R^4$, m, n, and s are as defined in claim 1.

**16.** The compound represented by formula (II-C-1) or (II-C-2) or the pharmaceutically acceptable salt thereof according to claim 15, wherein:

$R^1$ is hydrogen;
each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, and the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$;
$R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3;
preferably,
each $R^3$ is independently selected from the group consisting of fluorine, chlorine, cyano, methyl, trifluoromethyl, and trifluoromethoxy;
each $R^4$ is independently selected from the group consisting of fluorine, chlorine, cyano, methyl, trifluoromethyl, trifluoromethoxy, cyclopropyl, and cyanomethyl;
m is 0;
n and s are each independently selected from the group consisting of 0, 1, 2, and 3.

**17.** The compound represented by formula (II-C-3) or (II-C-4) or the pharmaceutically acceptable salt thereof according to claim 15, wherein:

$R^1$ is hydrogen;

each $R^2$ is independently selected from the group consisting of halogen, $C_{1-6}$ alkyl, $C_{1-6}$ alkoxy, $C_{1-6}$ haloalkyl, $C_{1-6}$ haloalkoxy, and 3- to 6-membered cycloalkyl, and the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^A$; or two $R^2$ attached to the same carbon atom, together with the carbon atom to which they are attached, form 3- to 6-membered cycloalkyl, and the 3- to 6-membered cycloalkyl is optionally substituted with one or more $R^A$;
$R^A$ is selected from the group consisting of halogen, hydroxy, cyano, nitro, amino, carboxyl, $C_{1-6}$ alkyl, $C_{1-6}$ alkoxy, $C_{1-6}$ haloalkyl, and $C_{1-6}$ hydroxyalkyl;
each $R^3$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the $C_{1-6}$ alkyl, $C_{1-6}$ alkoxy, $C_{1-6}$ haloalkyl, $C_{1-6}$ haloalkoxy, or 3- to 6-membered cycloalkyl is optionally substituted with one or more $R^B$, and $R^B$ is selected from the group consisting of halogen, hydroxy, and cyano;
each $R^4$ is independently selected from the group consisting of halogen, cyano, $C_{1-6}$ alkyl, $C_{1-6}$ alkoxy, $C_{1-6}$ haloalkyl, $C_{1-6}$ haloalkoxy, and 3- to 6-membered cycloalkyl, wherein the C1-6 alkyl, C1-6 alkoxy, C1-6 haloalkyl, C1-6 haloalkoxy, or 3- to 6-membered cycloalkyl is optionally substituted with one or more $R^C$, and $R^C$ is selected from the group consisting of halogen, hydroxy, and cyano;
m, n, and s are each independently selected from the group consisting of 0, 1, 2, and 3;
preferably,
each $R^2$ is independently selected from the group consisting of fluorine and methyl; or
two $R^2$ attached to the same carbon atom, together with the carbon atom to which they are attached, form cyclopropyl, wherein the cyclopropyl is optionally substituted with one or more $R^A$, and $R^A$ is a halogen;
each $R^3$ is independently selected from the group consisting of fluorine, chlorine, cyano, methyl, trifluoromethyl, and trifluoromethoxy;
each $R^4$ is independently selected from the group consisting of fluorine, chlorine, cyano, methyl, trifluoromethyl, trifluoromethoxy, cyclopropyl, and cyanomethyl;
m, n, and s are each independently selected from the group consisting of 0, 1, 2, and 3.

**18.** The compound or the pharmaceutically acceptable salt thereof according to any one of claims 1 to 17, being the following compounds or pharmaceutically acceptable salts thereof:

group A

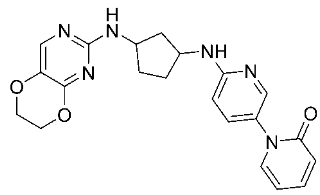 , 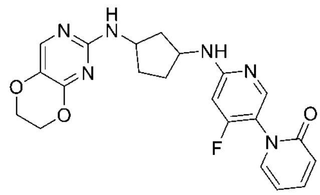 , 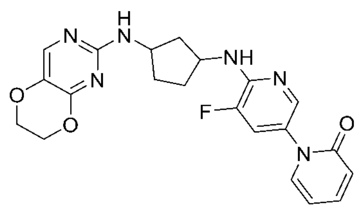 ,

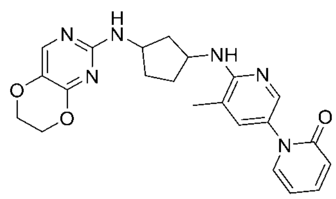 , 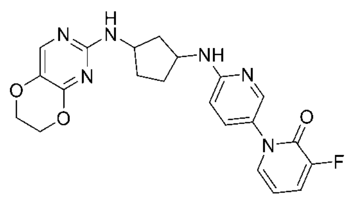 , 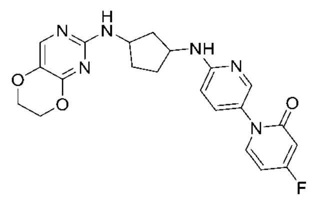 ,

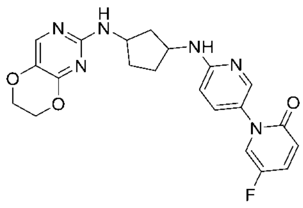, 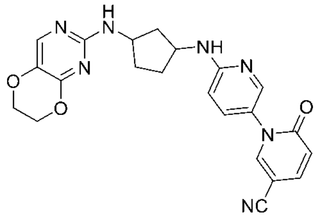, 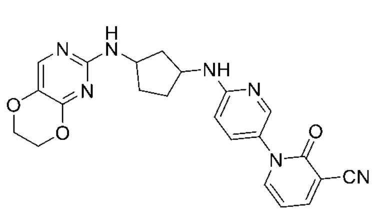,

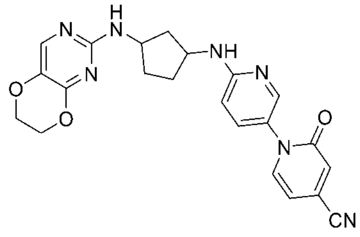, 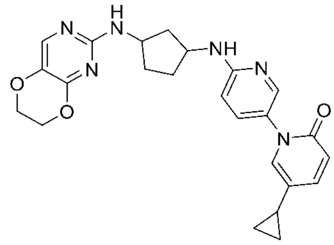, 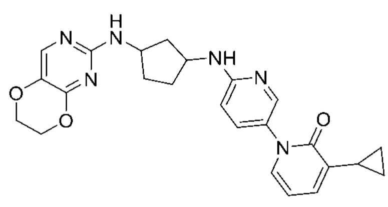,

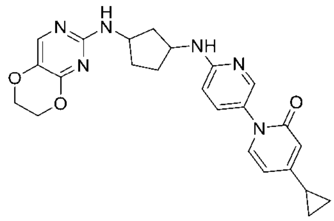;

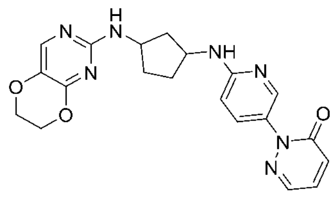, 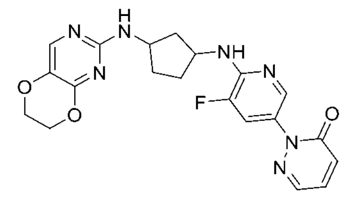, 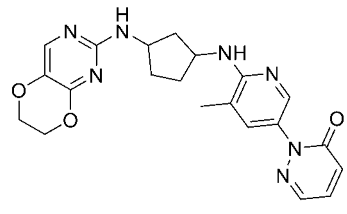,

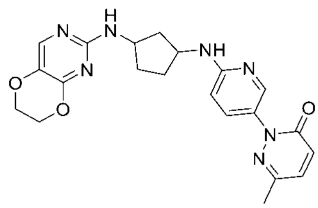, 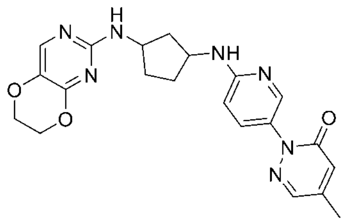, 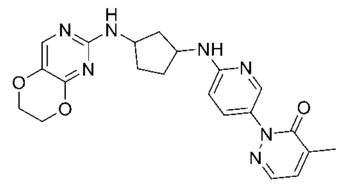;

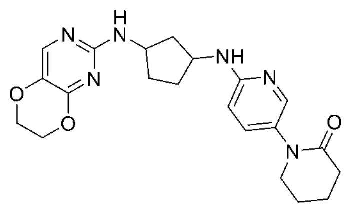, 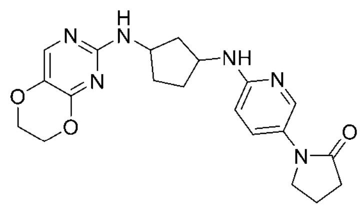, 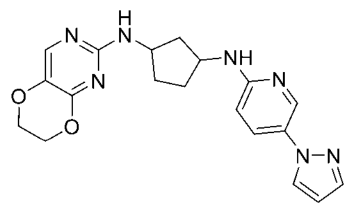;

group B

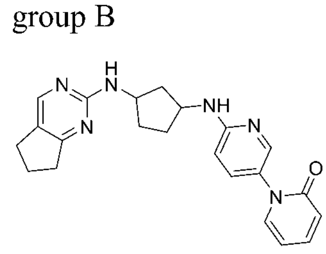, 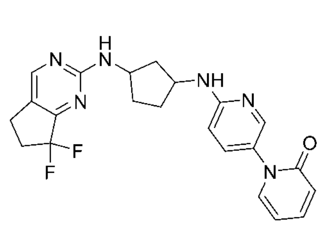, 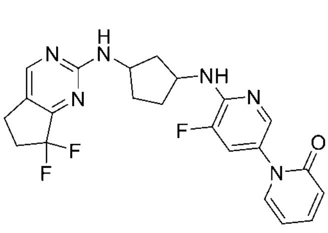,

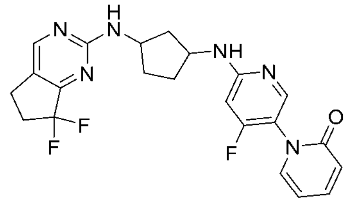, 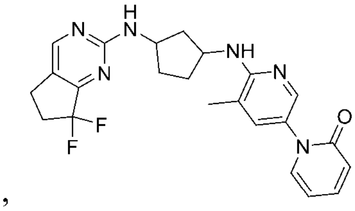, 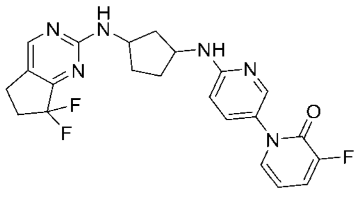,

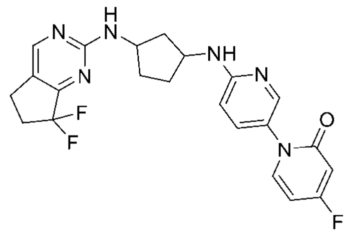, 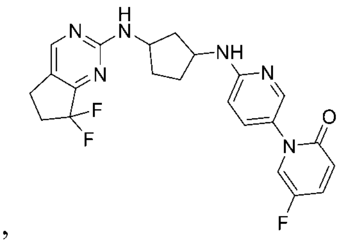, 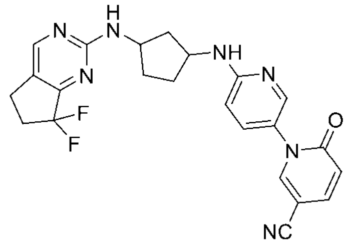,

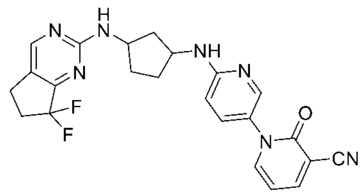, 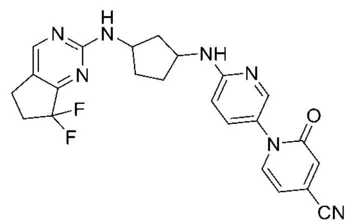, 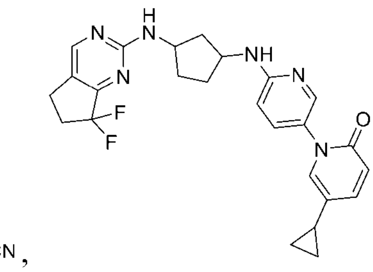,

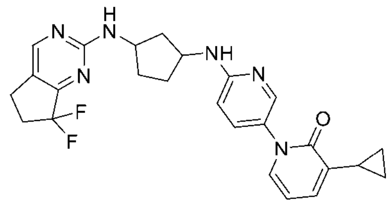, 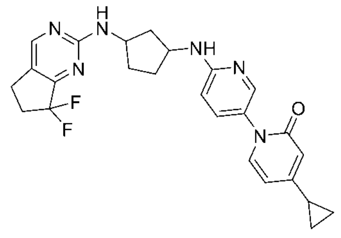, 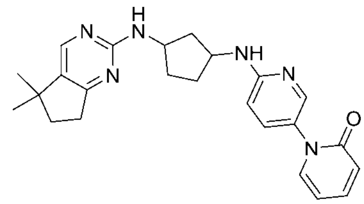,

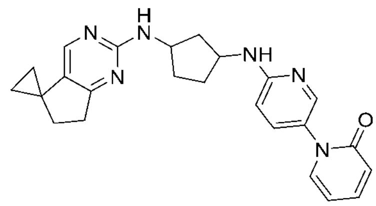, 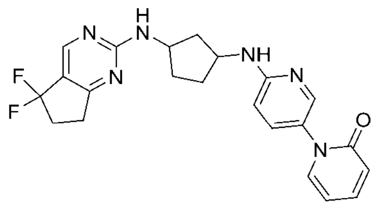;

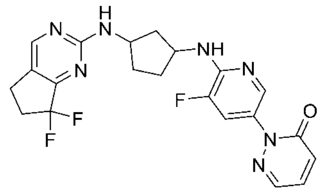, 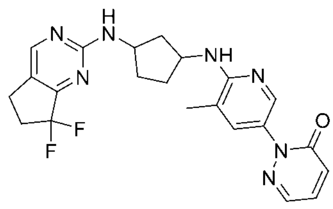, 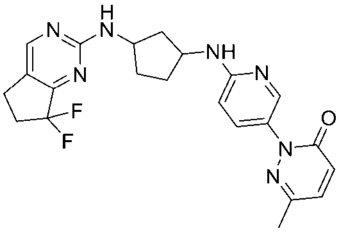,

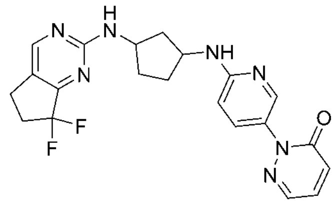, 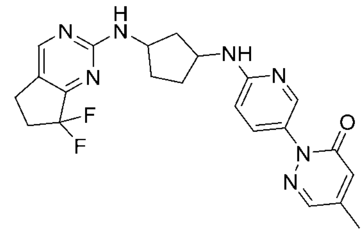, 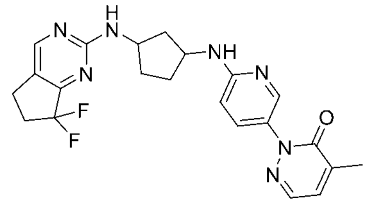,

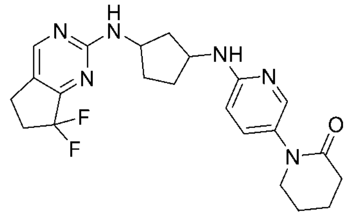,
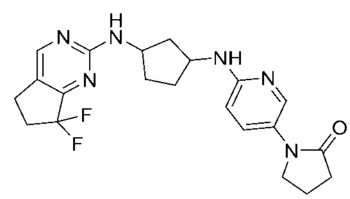,
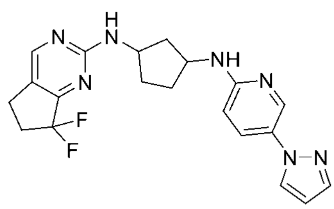;

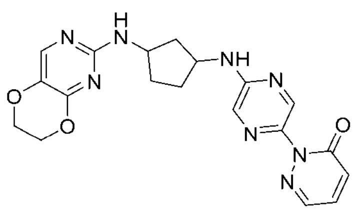,
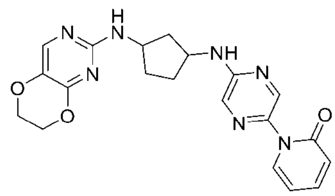,
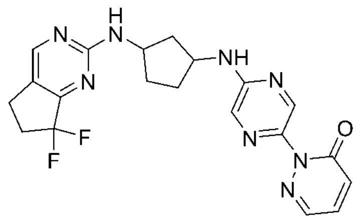,

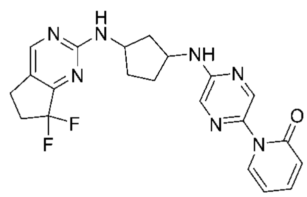,
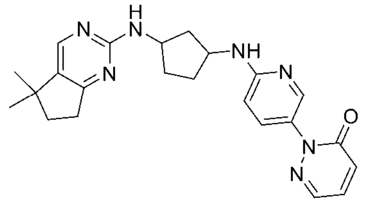,
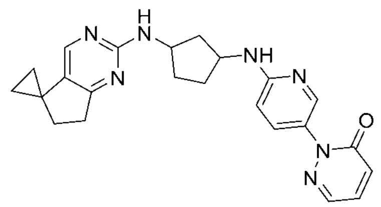,

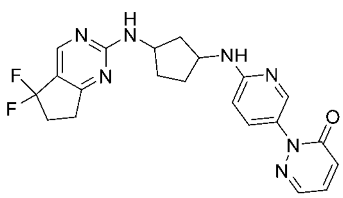,
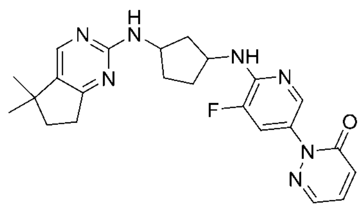,
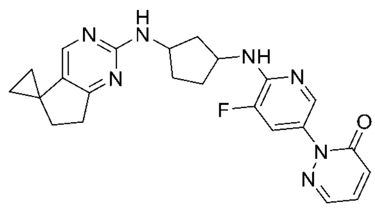,

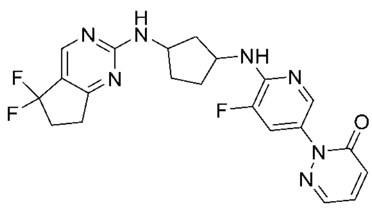;

group C

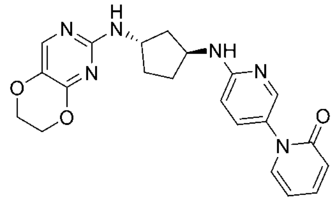, 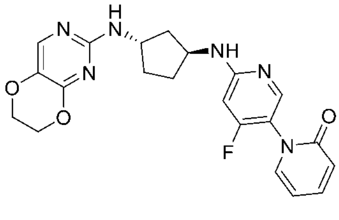, 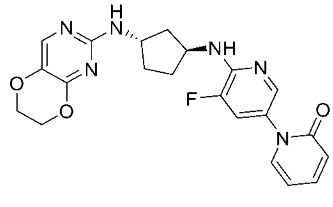,

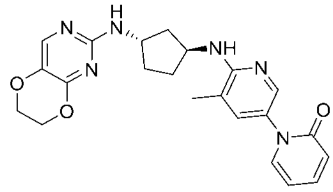, 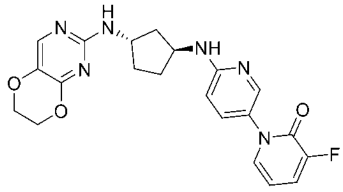, 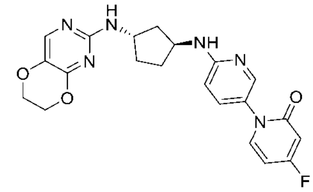,

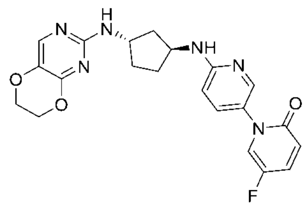, 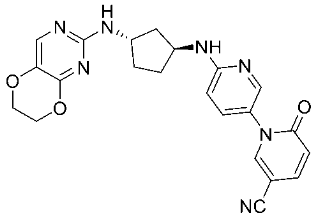, 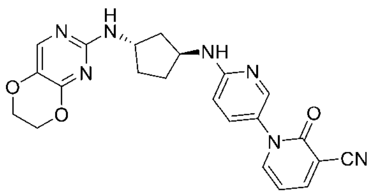,

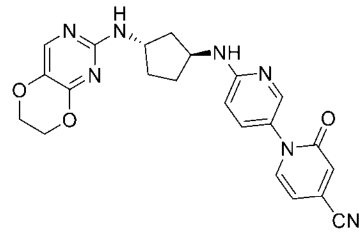, 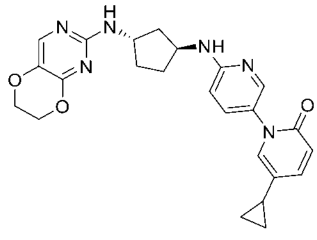, 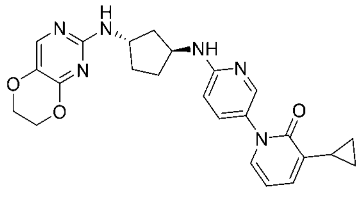,

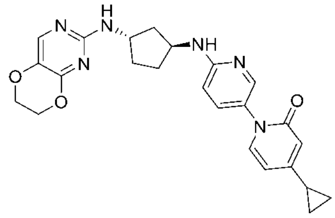;

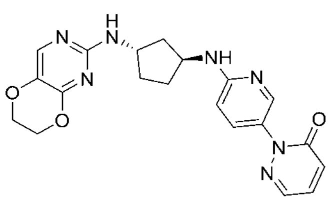, 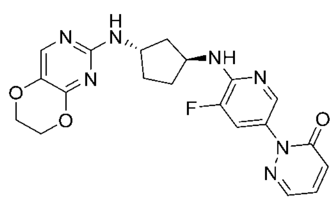, 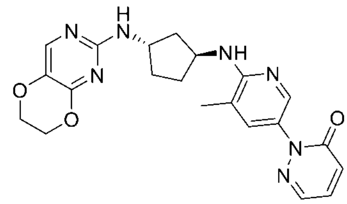,

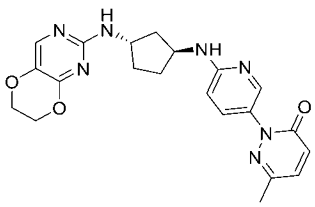, 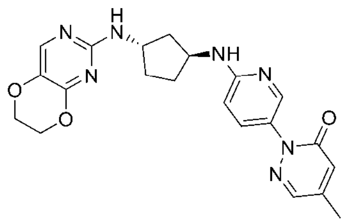, 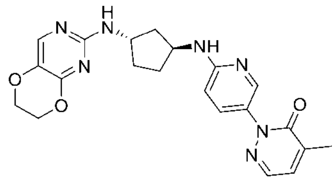;

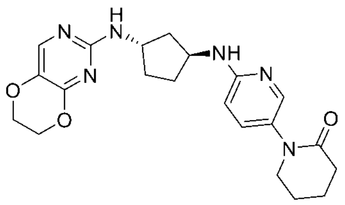, 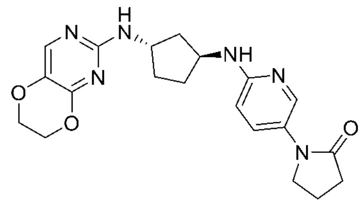, 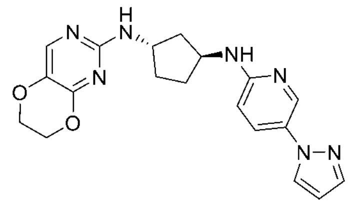;

group D

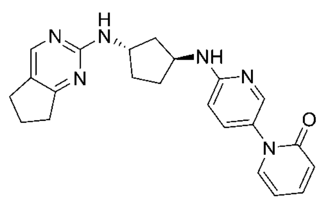, 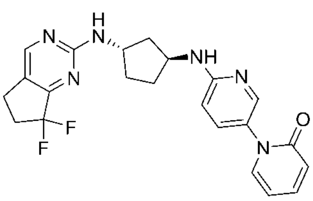, 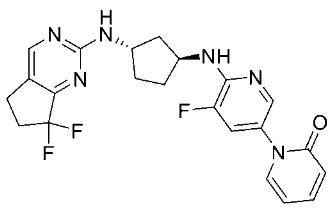,

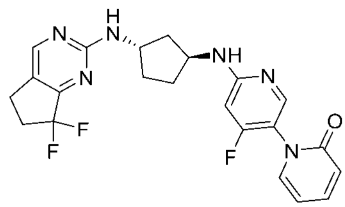, 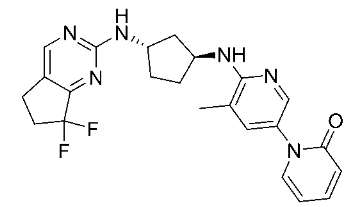, 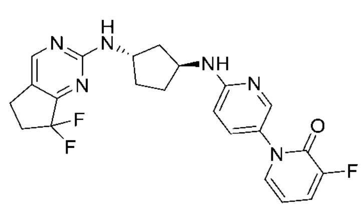,

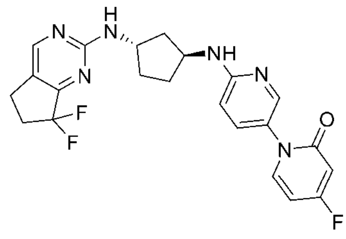,
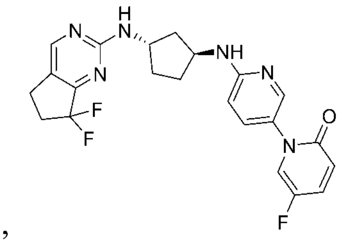,
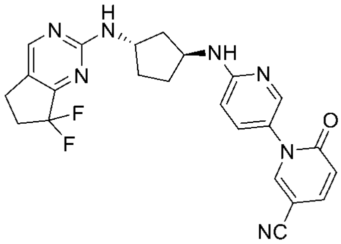,

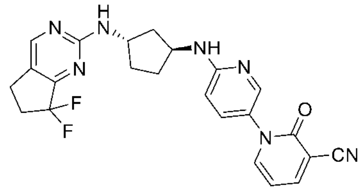,
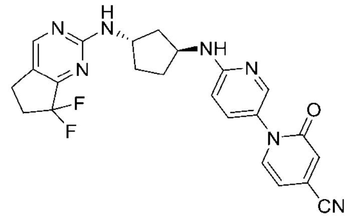,
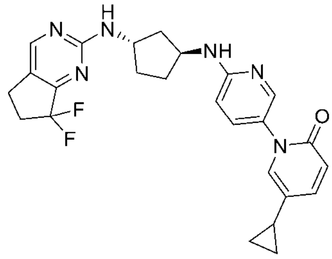,

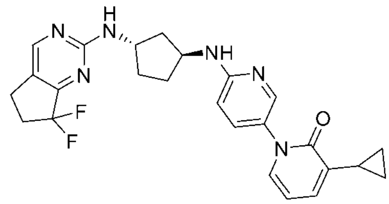,
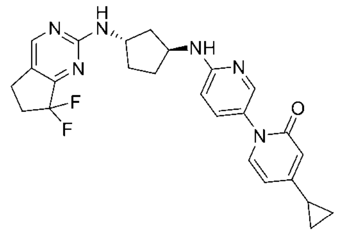,
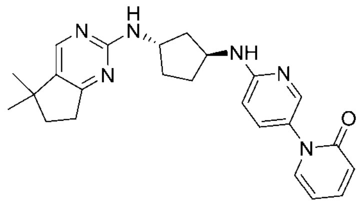,

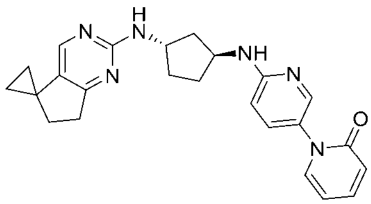,
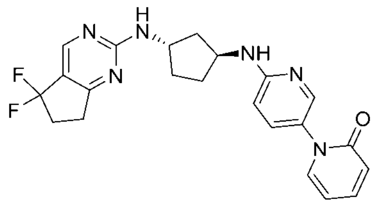;

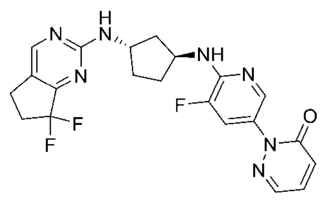,
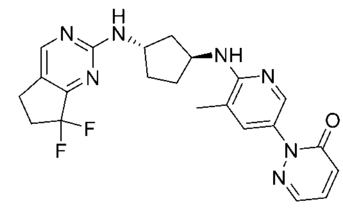,
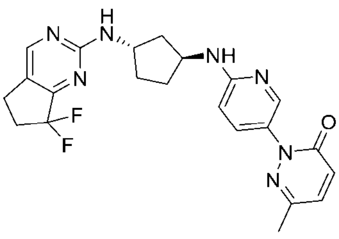,

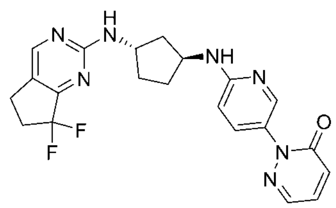,
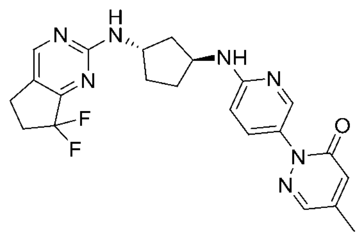,
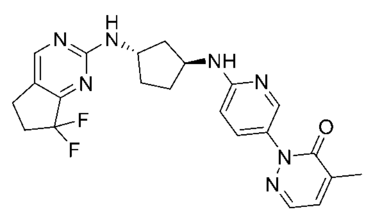,

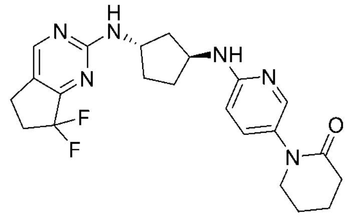,
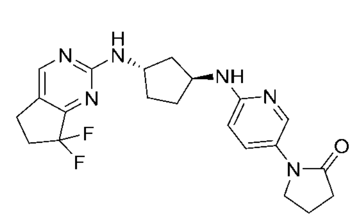,
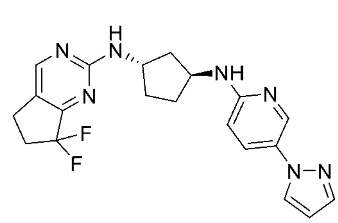;

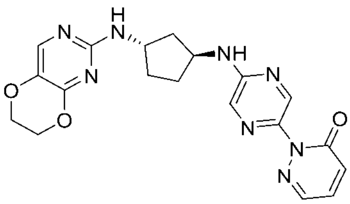, 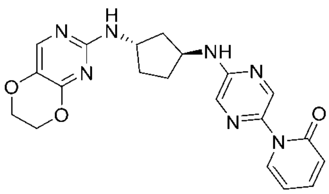, 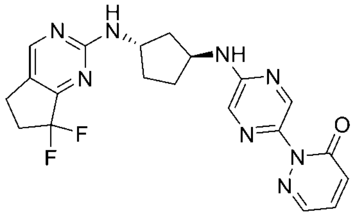,

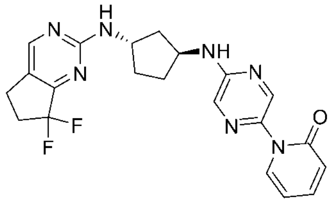, 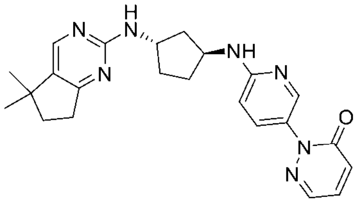, 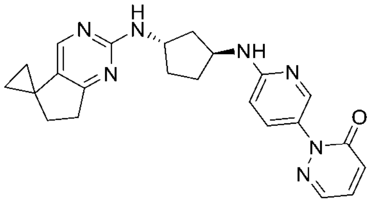,

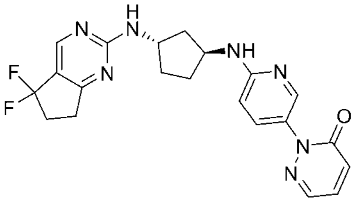, 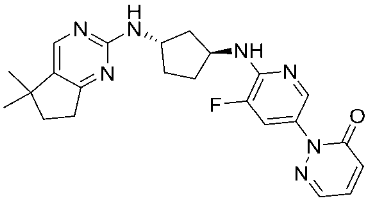, 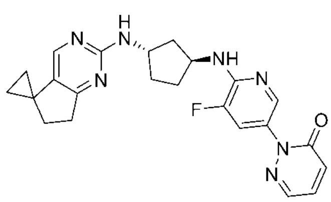,

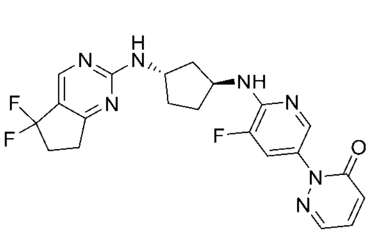, and 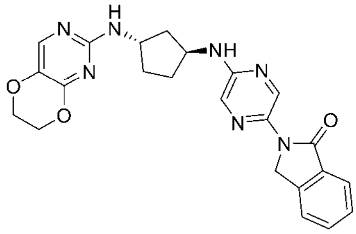.

**19.** An isotopically substituted form of the compound or the pharmaceutically acceptable salt thereof according to any one of claims 1 to 18, wherein the isotopically substituted form is a deuterated form.

**20.** A pharmaceutical composition, comprising the compound or the pharmaceutically acceptable salt thereof according to any one of claims 1 to 18 or the isotopically substituted form according to claim 19, and at least one pharmaceutically acceptable excipient.

**21.** Use of the compound or the pharmaceutically acceptable salt thereof according to any one of claims 1 to 18, or the isotopically substituted form according to claim 19, or the pharmaceutical composition according to claim 20, in the manufacture of a medicament for preventing and/or treating a disease and a disorder, wherein the disease and the disorder are selected from the group consisting of dyslipidemia, dyslipoproteinemia, hypercholesterolemia, hyperlipidemia, hypertriglyceridemia, hyperlipoproteinemia, xanthoma, hypoalphalipoproteinemia, sitosterolemia, atherosclerosis, arteriosclerosis, metabolic syndrome, coronary heart disease, peripheral vascular disease, congestive heart failure, stroke, vascular dementia, coronary artery disease, chronic kidney disease, retinopathy, inflammation, diabetic complications, and thrombi.

**22.** Use of the compound or the pharmaceutically acceptable salt thereof according to any one of claims 1 to 18, or the isotopically substituted form according to claim 19, or the pharmaceutical composition according to claim 20, in the manufacture of a medicament of a PCSK9 inhibitor.

**23.** Use of the compound or the pharmaceutically acceptable salt thereof according to any one of claims 1 to 18, or the isotopically substituted form according to claim 19, or the pharmaceutical composition according to claim 20, in the manufacture of a medicament for preventing and/or treating a PCSK9-mediated disease and a PCSK9-mediated disorder, wherein preferably, the PCSK9-mediated disease and the PCSK9-mediated disorder are selected from the group consisting of dyslipidemia, dyslipoproteinemia, hypercholesterolemia, hyperlipidemia, hypertriglyceridemia, hyperlipoproteinemia, xanthoma, hypoalphalipoproteinemia, sitosterolemia, atherosclerosis, arteriosclerosis, metabolic syndrome, coronary heart disease, peripheral vascular disease, congestive heart failure, stroke, vascular

dementia, coronary artery disease, chronic kidney disease, retinopathy, inflammation, diabetic complications, and thrombi.

**24.** A preparation method for the compound or the pharmaceutically acceptable salt thereof according to any one of claims 1 to 18, comprising a step of reacting a compound represented by formula (I-a) with a compound represented by formula (I-b) under alkaline conditions, optionally in the presence of a catalyst,

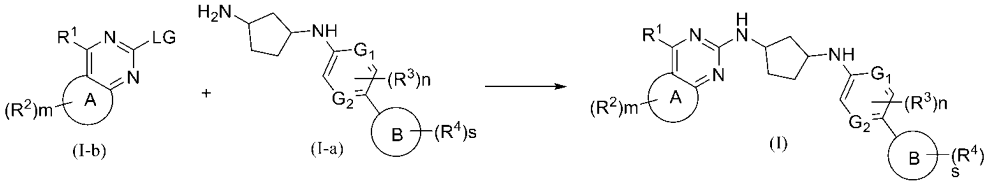

wherein LG is selected from the group consisting of halogen and $-S(O)_2R^7$, and $R^7$ is selected from the group consisting of $C_{1-6}$ alkyl and $C_{1-6}$ haloalkyl; and
$R^1$ , $R^2$, $G_1$, $G_2$, $R^3$, $R^4$, ring A, ring B, m, n, and s are as defined in claim 1.

**25.** A compound represented by formula (I-b),

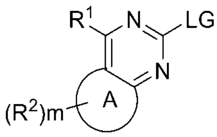

(I-b)

wherein:
LG is selected from the group consisting of halogen and $-S(O)_2R^7$, and $R^7$ is selected from the group consisting of $C_{1-6}$ alkyl and $C_{1-6}$ haloalkyl; $R^1$, $R^2$, and m are as defined in claim 1.

**INTERNATIONAL SEARCH REPORT**

International application No.
**PCT/CN2024/107781**

**A. CLASSIFICATION OF SUBJECT MATTER**

C07D491/056(2006.01)i; C07D471/04(2006.01)i; C07D498/04(2006.01)i; C07D401/02(2006.01)i; C07D417/12(2006.01)i; C07D487/04(2006.01)i; C07D401/12(2006.01)i; C07D403/12(2006.01)i; A61K31/519(2006.01)i; A61K31/444(2006.01)i; A61K31/501(2006.01)i; A61K31/505(2006.01)i; A61K31/4745(2006.01)i; A61P9/00(2006.01)i; A61P3/06(2006.01)i; A61P1/16(2006.01)i; A61P9/10(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXTC, WPABS, WPABSC, STN (REGISTRY, CAPLUS, MARPAT): 上海拓界生物, 胡涛, 谭亮, 刘浩淼, 李云飞, 蛋白原转化酶枯草杆菌蛋白酶/kexin 9, 心血管, 高胆固醇血症, 高脂血症, 高脂蛋白血症, TUOJIE BIOTECH, PCSK9, cardiovascular, hypercholesterolemia, hyperlipidemia, hyperlipoproteinemia

**C. DOCUMENTS CONSIDERED TO BE RELEVANT**

| Category* | Citation of document, with indication, where appropriate, of the relevant passages | Relevant to claim No. |
|---|---|---|
| PX | WO 2024078620 A1 (SHANGHAI HANSOH BIOMEDICAL CO., LTD. et al.) 18 April 2024 (2024-04-18)<br>claims 1-18 | 1-25 |
| X | REGISTRY. "RN 2839408-77-2 etc."<br>*STN*, 12 October 2022 (2022-10-12),<br>pp. 1-10 | 25 |
| A | WO 2020150473 A2 (DOGMA THERAPEUTICS, INC.) 23 July 2020 (2020-07-23)<br>claims 1-53 | 1-25 |
| A | WO 2020150474 A1 (DOGMA THERAPEUTICS, INC.) 23 July 2020 (2020-07-23)<br>claims 20-73 | 1-25 |
| A | WO 2023084449 A1 (NOVARTIS AG) 19 May 2023 (2023-05-19)<br>claims 1-40 and 54-56 | 1-25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 October 2024** | **01 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/107781**

**C. DOCUMENTS CONSIDERED TO BE RELEVANT**

| Category* | Citation of document, with indication, where appropriate, of the relevant passages | Relevant to claim No. |
|---|---|---|
| A | WO 2014150326 A1 (SHIFA BIOMEDICAL CORPORATIION) 25 September 2014 (2014-09-25)<br>claims 1-5 | 1-25 |
| A | WO 2020252383 A2 (SRX CARDIO, LLC) 17 December 2020 (2020-12-17)<br>claims 1-25 | 1-25 |
| A | WO 2022133529 A1 (CARDIO THERAPEUTICS PTY. LTD.) 30 June 2022 (2022-06-30)<br>claims 1-26 | 1-25 |

Form PCT/ISA/210 (second sheet) (July 2022)

**INTERNATIONAL SEARCH REPORT**

International application No.
**PCT/CN2024/107781**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **25**
because they relate to parts of the international application that do not comply with the prescribed requirements to such an extent that no meaningful international search can be carried out, specifically:

The compound of the general formula in claim 25 covers a large number of compounds of different structures, and therefore no effective search can be performed. The present search report is provided on the basis of the specific compound of claim 18.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/107781**

| Patent document cited in search report | Publication date (day/month/year) | Patent family member(s) | Publication date (day/month/year) |
|---|---|---|---|
| WO 2024078620 A1 | 18 April 2024 | TW 202423430 A | 16 June 2024 |
| WO 2020150473 A2 | 23 July 2020 | CL 2021001859 A1 | 14 January 2022 |
| | | PE 20220138 A1 | 27 January 2022 |
| | | CA 3125765 A1 | 23 July 2020 |
| | | DOP 2021000150 A | 31 August 2021 |
| | | US 2022220122 A1 | 14 July 2022 |
| | | ECSP 21060240 A | 30 September 2021 |
| | | EP 3911648 A2 | 24 November 2021 |
| | | EP 3911648 A4 | 26 October 2022 |
| | | SG 11202107614 PA | 30 August 2021 |
| | | JOP 20210193 A1 | 30 January 2023 |
| | | CO 2021010312 A2 | 20 September 2021 |
| | | US 2020291041 A1 | 17 September 2020 |
| | | US 11248001 B2 | 15 February 2022 |
| | | MX 2021008533 A | 19 August 2021 |
| | | JP 2022518015 A | 11 March 2022 |
| | | JP 7128969 B2 | 31 August 2022 |
| | | AU 2020209215 A1 | 26 August 2021 |
| | | AU 2020209215 B2 | 02 February 2023 |
| | | KR 20210116548 A | 27 September 2021 |
| | | MA 54261 A | 27 April 2022 |
| | | BR 112021013807 A2 | 30 November 2021 |
| | | TW 202043213 A | 01 December 2020 |
| | | IL 284640 A | 31 August 2021 |
| | | JP 2022116083 A | 09 August 2022 |
| | | EA 202191892 A1 | 24 February 2022 |
| | | WO 2020150473 A3 | 04 February 2021 |
| | | CR 20210441 A | 11 March 2022 |
| WO 2020150474 A1 | 23 July 2020 | KR 20210116549 A | 27 September 2021 |
| | | MX 2024009001 A | 31 July 2024 |
| | | CA 3125767 A1 | 23 July 2020 |
| | | TW 202042817 A | 01 December 2020 |
| | | AU 2023201972 A1 | 04 May 2023 |
| | | MA 54758 A | 27 April 2022 |
| | | EP 3911640 A1 | 24 November 2021 |
| | | EP 3911640 A4 | 26 October 2022 |
| | | MX 2021008661 A | 19 August 2021 |
| | | US 2023407285 A1 | 21 December 2023 |
| | | SG 11202107615 TA | 30 August 2021 |
| | | IL 284661 A | 31 August 2021 |
| | | JP 2022518016 A | 11 March 2022 |
| | | US 2020231584 A1 | 23 July 2020 |
| | | US 11603523 B2 | 14 March 2023 |
| | | AU 2020209216 A1 | 26 August 2021 |
| | | EA 202191890 A1 | 03 February 2022 |
| | | BR 112021013924 A2 | 21 September 2021 |
| WO 2023084449 A1 | 19 May 2023 | TW 202333563 A | 01 September 2023 |
| WO 2014150326 A1 | 25 September 2014 | JP 2019031522 A | 28 February 2019 |
| | | JP 6691948 B2 | 13 May 2020 |
| | | AU 2014237312 A1 | 05 November 2015 |

Form PCT/ISA/210 (patent family annex) (July 2022)

**INTERNATIONAL SEARCH REPORT**
**Information on patent family members**

International application No.
**PCT/CN2024/107781**

| Patent document cited in search report | Publication date (day/month/year) | Patent family member(s) | Publication date (day/month/year) |
|---|---|---|---|
| | | AU 2014237312 B2 | 28 March 2019 |
| | | CA 2904660 A1 | 25 September 2014 |
| | | CA 2904660 C | 06 April 2021 |
| | | EP 2968266 A1 | 20 January 2016 |
| | | EP 2968266 A4 | 07 September 2016 |
| | | EP 2968266 B1 | 01 May 2019 |
| | | JP 2016512825 A | 09 May 2016 |
| | | US 2016256467 A1 | 08 September 2016 |
| | | BR 112015023294 A2 | 18 July 2017 |
| WO 2020252383 A2 | 17 December 2020 | US 2022267269 A1 | 25 August 2022 |
| | | CA 3142890 A1 | 17 December 2020 |
| | | AU 2020291468 A1 | 06 January 2022 |
| | | JP 2022537140 A | 24 August 2022 |
| | | EP 3983392 A2 | 20 April 2022 |
| | | EP 3983392 A4 | 06 December 2023 |
| | | WO 2020252383 A3 | 18 March 2021 |
| | | IL 288848 A | 01 February 2022 |
| WO 2022133529 A1 | 30 June 2022 | AU 2021407529 A1 | 20 July 2023 |
| | | US 2024124428 A1 | 18 April 2024 |
| | | EP 4263520 A1 | 25 October 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023084449 A
- WO 2020150473 A
- WO 2020150474 A
- WO 201528848 A